# EUROPEAN PATENT APPLICATION

(11) **EP 2 014 651 A1**
(43) Date of publication of application: **14.01.2009**
(21) Application number: 07301230.4
(22) Date of filing: 12.07.2007
(51) Int. Cl.: C07D 217/10, C07D 217/20, A61K 31/4375, A61K 31/47, A61K 31/472, A61K 31/473, A61P 7/02, A61P 9/10, A61P 9/12, A61P 25/28, A61P 27/02, A61P 35/00

(54) **Compounds and methods for modulating Rho GTPases**

(71) Applicant: Exonhit Therapeutics SA, 75017 Paris (FR)
(72) Inventor: Leblond, Bertrand, 75011, PARIS (FR); Beausoleil, Eric, 75015, PARIS (FR); Chauvignac, Cédric, 92120, MONTROUGE (FR); Taverne, Thierry, 62280, SAINT MARTIN BOULOGNE-SUR-MER (FR); Picard, Virginie, 75011, Paris (FR); De Oliveira, Catherine, 94320, THIAIS (FR); Schweighoffer, Fabien, 94120, FONTENAY SOUS BOIS (FR)
(74) Representative: Tezier Herman, Béatrice

(57) **Abstract**

The present invention relates to methods and compositions that affect the GTP-binding activity of members of the Rho family GTPases, preferably Rac GTPases (Rac1, Raclb, Rac2 and/or Rac3).

## Description

The present invention relates to methods and compositions that affect the GTP-binding activity of members of the Rho family GTPases, preferably Rac GTPases (Rac1, Raclb, Rac2 and/or Rac3).

Rho family GTPases are molecular switches that control signalling pathways regulating cytoskeleton reorganization, gene expression, cell cycle progression, cell survival, and other cellular processes (Etienne-Manneville S., and Hall A., 2002, Nature, 420, 629-635, which is incorporated herein by reference in its entirety).

Rho GTPases of the Ras superfamily are involved in the regulation of multiple cell functions and have been implicated in the pathology of various human diseases including cancers (Fritz G., Just I., and Kaina B., Int. J. Cancer, 1999, 81, 682-687; Fritz G., Kaina B. Curr. Cancer Drug Targets, 2006, 6, 1-14; Sahai E., Marshall C.J., Nat. Rev. Cancer., 2002, 2, 133-42), pathological angiogenesis such as in diabetic retinopathy, tumoral angiogenesis, glaucoma, age-related macular degeneration (Eriksson A., Cao R., Roy J., Tritsaris K., Wahlestedt C., Dissing S., Thyberg J., Cao Y., Circulation, 2003, 107, 1532-8; Soga N., Namba N., McAllister S., Cornelius L., Teitelbaum S.L., Dowdy S.F., Kawamura J., Hruska K.A., Exp. Cell. Res., 2001, 269, 73-87; Fryer B.H., Field J., Cancer Lett., 2005, 229, 13-23), asthma, Alzheimer's disease (Désiré L., Bourdin J., Loiseau N., Peillon H., Picard V., De Oliveira C., Bachelot F., Leblond B., Taverne T., Beausoleil E., Lacombe S., Drouin D., Schweighoffer F., J. Biol. Chem., 2005, 280(45), 37516-25), cardiac left ventricular hypertrophy (Brown J.H., Del Re D.P., Sussman M.A., Circ Res., 2006, 98, 730-42; Molkentin J.D., Dorn II G.W., 2nd Annu Rev Physiol. 2001, 63, 391-426). They are attractive drug targets in future targeted therapy (Nassar N., Cancelas J., Zheng J., Williams D.A., and Zheng Yi, Current topics in Medicinal Chemistry, 2006, 6, 1109-1116).

Rho family proteins constitute one of three major branches of the Ras superfamily. Rho proteins share approximately 30 percent amino acid identity with the Ras superfamily proteins. At least 14 mammalian Rho family proteins have been identified so far, including RhoA, RhoB, RhoC, RhoE/Rnd3, Rnd1/Rho6, Rnd2/Rho7, RhoG, Rac1, Raclb, Rac2, Rac3, Cdc42, TC10, and TTF.

Rac proteins (Rac1, 1b, 2, 3) belong to the Rho GTP-binding proteins (or GTPases) of the Ras superfamily and thus act as molecular switches cycling between an active GTP-bound and an inactive GDP-bound form through nucleotide exchange and hydrolysis. Like most other GTPases, these proteins adopt different conformations depending on the bound nucleotide, the main differences lying in the conformation of two short and flexible loop structures designated as the switch I and switch II regions. The three distinct mammalian Rac isoforms, Rac1, 2 and 3, share a very high sequence identity (up to 90 %), with Raclb being an alternative splice variant of Rac1 with a 19 amino acid insertion in vicinity to the switch II region. Raclb has an accelerated GEF-independent GDP/GTP-exchange and an impaired GTP-hydrolysis, accounting for a self-activating GTPase (Haeusler L.C. et al., Methods in Enzymology, 2006, 406, 1-11).

Rac1 regulates the activity of the superoxide anion generating NADPH oxidase system of phagocytes, plays a central role in organization of the actin cytoskeleton, and is essential for Ras-induced transformation. In addition, mutant, constitutively active Raclb can induce cellular transformation, invasion, and metastasis. Similar to Ras proteins, Rac1 is activated by upstream GEFs (Guanine nucleotide Exchange Factors) and binds effector proteins that signal downstream. Human cells contain 3 homologous Rac proteins, Rac1, Rac2, and Rac3, that are essentially identical except for the hypervariable C-terminal domains. Rac1, but not Rac2 or Rac3, contains a polybasic domain within its hypervariable region that is virtually identical to the polybasic domain of K-Ras 4B.

Rac1 binds to and activates the effector protein PAK1 far more efficiently than Rac2 does, and the polybasic domain of Rac1 directly accounts for the enhanced ability of Rac1 to bind to and activate PAK1 (Knaus U.G., Wang Y., Reilly A.M., Warnock D., and Jackson J.H., J. Biol. Chem., 1998, 273, 21512). The polybasic domain is also crucial for Rac1 mediated activation of NADPH oxidase and membrane ruffling but is not required for Rac1 mediated cell transformation or binding of Rac1 to the effector protein POR1 (Jones M.K., and Jackson J.H., J. Biol. Chem., 1998, 273, 1782).

NSC 23766 described in international patent application WO 2007/016539 is a cell-permeable pyrimidine compound that specifically and reversibly inhibits Rac1 GDP/GTP exchange activity by interfering Rac1 interaction with the Rac-specific GEFs (guanine nucleotide exchange factor) Trio and Tiam1 (IC₅₀ ~ 50 µM). NSC 23766 inhibit Rac1-mediated cellular functions in NIH3T3 and PC-3 cells (effective dose ~50 to 100 µM). NSC 23766 exhibits no effect on Cdc42 or RhoA activation, nor does it affect Rac1 interaction with BcrGAP or PAK1 (Nassar N., Cancelas J., Zheng J., D. Williams A., and Zheng Yi, Current topics in Medicinal Chemistry, 2006, 6, 1109-1116).

EHT 1864 described in international patent application WO 2004/076445, is a small molecule that blocks the Rac1 signaling pathways. *In vitro,* EHT 1864 blocks Abeta 40 and Abeta 42 production but does not impact sAPPalpha levels and does not inhibit beta-secretase. Rather, EHT 1864 modulates APP processing at the level of gamma-secretase to prevent Abeta 40 and Abeta 42 generation. This effect does not result from a direct inhibition of the gamma-secretase activity and is specific for APP cleavage, since EHT 1864 does not affect Notch cleavage. *In vivo,* EHT 1864 significantly reduces Abeta 40 and Abeta 42 levels in guinea pig brains at a threshold that is compatible with delaying plaque accumulation and/or clearing the existing plaque in brain. EHT 1864 was the first derivative of a new chemical series that consists of candidates for inhibiting Abeta formation in the brain of Alzheimer patients as described in US patent No. 2007/0027146 (compound 38). EHT 1864 represented the first pharmacological validation of Rac1 signaling as a target for developing novel therapies for Alzheimer's disease (Désiré L., Bourdin J., Loiseau N., Peillon H., Picard V., De Oliveira C., Bachelot F., Leblond B., Taverne T., Beausoleil E., Lacombe S., Drouin D., and Schweighoffer F., J. Biol. Chem., 280 (45), 2005, 37516-25).

Berberine is a member of the protoberberine class of isoquinoline alkaloids. It is probably the most widely distributed of all alkaloids, having been found in the roots, rhizomes, and stem bark of the plants of nine botanical families, Berberidaceae, Papaveraceae, Rununculaceae, Rutaceae, Menispermaceae, Rubiaceae, Rhamnaceae, Magnoliaceae, and Annonaceae.

Other known members of the protoberberine class of isoquinoline alkaloids are jatrorrhizine chloride, columbamine chloride, berberrubine chloride, thalifendine chloride, coptisine chloride and nandinine hydrochloride, etc...

The protoberberine alkaloids display a broad diversity of biological activities (Simeon S., Rios J.L., and Villar A., Plant Med. Phytother., 1989, 23, 202; Bhakuni D.A., and Jain S., The Alkaloids, Academic Press, 1986, 28, 95; Shamma M., and Moniot J.L., Isoquinoline Alkaloids Research 1972-1977, Plenum Press, New York & London, 1978, 209; Shamma M., The Isoquinoline Alkaloids, Chemistry and Pharmacology, Academic Press, New York & London, 1972, 268; Kondo Y., Heterocycles, 1976, 4, 197) and feature predominently as active components in many folkloric medicines (Thakur, R.S., Srivastava, S.K., Cent. Inst. Med. Aromatic Plants, 1982, 4, 249) especially in Native America, China and other Asian countries.

In traditional practices of Ayurvedic and Chinese medicine, numerous plants have been used to treat cognitive disorders, including neurodegenerative diseases such as Alzheimer's disease (AD). *Coptis chinensis* (Rununculaceae) has been used in traditional Chinese medicine for several conditions. A methanol extract fraction of *C. chinensis,* jatrorrhizine and berberine are MAO inhibitors (Kong L.D., Cheng, C.H. and Tan R.X., Planta Med., 2001, 67(1), 74-76), indicating potential antidepressant activity, and C. *chinensis* and some alkaloids isolated from this plant (berberine, coptisine and palmatine) are reported to be anti-ChE (Huang K.C., CRC Press, Boca Raton (FL) 1993; Park C.H., Kim S., Choi W., Lee Y., Kim J., Kang S.S. et al., Planta Med., 1996, 62, 405-409 and Shigeta K., Ootaki K., Tatemoto H., Nakanishi T., Inada A., and Muto, N., Biosci. Biotechnol. Biochem., 2002, 66(11), 2491-2494). C. *chinensis* has also shown anti-inflammatory (Cuéllar M.J., Giner R.M., Recio M.C., Máñez S., and Rios J.L., Fitoterapia, 2001, 72(3), 221-229) and antioxidant activities (Liu F., and Ng T.B., Life Sci., 2000, 66(8), 725-735 and Schinella G.R., Tournier H.A., Prieto J.M. Mordujovich de Buschiazzo P., and Rios J.L., Life Sci., 2002, 70, 1023-1033) and it improved a scopolamine-induced learning and memory deficit in rats (Hsieh M.T., Peng W.H., Wu C.R., and Wang W.H., Phytother. Res., 2000., 14(5), 375-377). As well as inhibiting AChE, the alkaloids coptisine, palmatine and berberine in particular, also showed NGF-enhancing activity in PC12 cells (Shigeta K., Ootaki K., Tatemoto H., Nakanishi T., Inada A., and Muto N., Biosci. Biotechnol. Biochem., 2002, 66(11), 2491-2494).

Other pharmacological properties include antimicrobial, antimalarial, antileukemic, antiulcerous, gastric antisecretory, and enzyme inhibitory activities.

The mechanism of antimicrobial activity of berberine is related to its effect on DNA intercalation and inhibition of reverse transcription and DNA synthesis in microorganism cells. Berberine has an antimicrobial activity against a variety of organisms including bacteria, viruses, fungi, protozoans, helminths, and chlamydia. Currently, the predominant clinical uses of berberine include bacterial diarrhea, intestinal parasite infections and treatment of infected eyes and eye irritations (Murine^{™}).

The cytotoxicity of several protoberberine alkaloids against human cancer cell line (lung, colon, CNS, stomach, ovarian, breast, renal, melanoma) was also investigated (Iwasa K., Moriyasu M., Yamori T., Turuo T., Lee D.-U., and Wiegrebe W., J. Nat. Prod., 2001, 64, 896-898). It was shown that the cytoxic activity paralleled the antimicrobial activity.

Berberine reduces total cholesterol, low-density lipoprotein (LDL) cholesterol, and triglycerides in both humans (at 1 g/day) and hamsters fed 50 mg/Kg/day along with a high fat diet. Berberine is therefore a natural product that may help control serum cholesterol without the side effects typical of the statin family of hypocholesterolemic drugs (Kong W., Wei J., Abidi P., et al., Nature Med., 2004, 10(12), 1344-1351).

Sources of benzo[c]phenanthridine alkaloids include five plant families: Papaveraceae, Fumariaceae, Rutaceae, Capitofoliaceace and Meliaceae. The most important source of benzo[c]phenanthridine alkaloids are found in the Papaveraceae plant family. Sanguinarine and chelerythrine are quarternary alkaloids isolated respectively from the root of *sanguinaria canadensis L.* and *Chelidonium majus L.* These alkaloids are known as sanguinaria extracts. Patents describing an extract of these alkaloids include USSR Pat. No. 495,311 and German Pat. No. 2,856,577. The benzo-[c]-phenanthridine alkaloids have valuable properties as antimicrobials as well as in treating mouth odors, gingivitis, and periodontitis. Extract of the plant has been used in toothpastes and oral rinse products (Kufrinec M.M., Mueller-Joseph L.J., Kopczyk R.A., J. Can. Dent. Assoc., 1990, 56, 31-35). Such alkaloids can be purchased commercially and/or isolated from plants as known in the art and as described, for example, in U.S. Pat. No. 5,133,981.

Other known members of the benzo[c]phenanthridine alkaloids are fagaronine, nitidine, oxysanguinarine, oxyavycine, oxynitidine, norsanguinarine, chelirubine, macarpine, 6-oxochelerythrine, 5,6-dihydrochelerythrine, norchelerythrine, etc...

Benzo[c]phenanthridine alkaloids are known to have anticancer properties. (Stermitz F. R., Gillespie J.P., Amoros L.G., Romero R., Stermitz T. A., Larson K. A., Earl S., and Ogg J. E., J. Med. Chem., 1975, 18(7), 708-713).

Changes in activation balance of different protein kinase C (PKC) isoenzymes have been linked to cancer development. Interestingly, sanguinarine was shown essentially inactive against PKC (217 µM) (Wang, B. H., Lu, Z. X., and Polya, G. M., Planta Med. 1997, 63, 494-498), whereas the closely related chelerythrine has been reported as a potent (1 µM) inhibitor of this kinase. Mitogen-activated protein kinase phosphatase-1 (MKP-1) is a dual specificity phosphatase that is overexpressed in many human tumors and can protect cells from apoptosis caused by DNA-damaging agents or cellular stress. Both chelerythrine and sanguinarine have MKP-1 inhibitory activity (Vogt A., Tamewitz A., Skoko J., Sikorski R.P., Giuliano K.A., and Lazo J.S., J. Biol. Chem., 2005, 280 (19), 19078-19086).

Sanguinarine is also known to possess interesting antiangiogenic properties (Giuseppina B. et al., Ann. N.Y. Acad. Sci., 2007, 1095, 371-376). This process impacts significantly on many important disease states including cancer, diabetic retinopathy, and arthritis.

Chelerythrine is also currently in development for the treatment of bipolar disorder and the cognitive deficits of schizophrenia. Chelerythrine's utility for treating CNS disorders, based on its PKC inhibition, was discovered by Amy Arnsten at Yale University (international patent application WO 2005/030143). Taken orally, chelerythrine has proven to be very potent in multiple models of memory disorders including a sophisticated primate model of prefrontal cortex-dependent working memory.

The present invention is based on the identification of the inhibitory action of particular protoberberine alkaloids and benzo[c]phenanthridine class of alkaloids on the activity of Rho family GTPases, in particular on the activity of the members of Rac subfamily of Rho GTPases.

Accordingly, the present invention relates to the use of protoberberine or benzo[c]phenanthridine alkaloids in *an in vitro* method for modulating, preferably inhibiting, a member of the Rho GTPase family.
The present invention further relates to the use of a compound of formula (I) or (II) as defined herein below for the manufacture of a pharmaceutical composition for treating a pathology involving a member of the Rho GTPase family.
The invention further relates to compounds of formula (I) or (II) as defined herein below and pharmaceutical compositions comprising the same.

It has been found that compounds of formula (I) and (II) described below, and pharmaceutically acceptable derivatives thereof, specifically inhibit Rho GTPases, in particular Rac GTPases, and can be effective in modulating Rho GTPases functions, in particular Rac-mediated functions, in diverse cellular systems including tumor cell transformation and invasion and hematopoietic stem/progenitor cell mobilization.

One object of the invention is thus to provide *an in vitro* method for modulating, preferably for inhibiting, a member of the Rho GTPase family, wherein the GTPase is contacted with a compound of formula (I) or (II), the compound of formula (I) having the following structure: 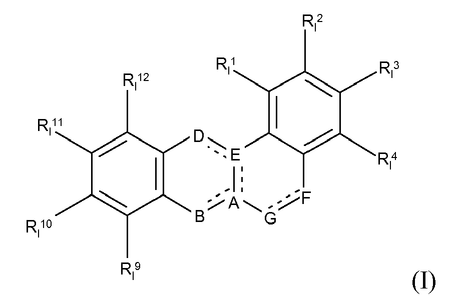 in which
R_{I}¹, R_{I}⁴ and R_{I}¹² independently represent H, a C₁-C₆ alkyl group, a C₂-C₆ alkenyl group or a C₂-C₆ alkynyl group;
R_{I}², R_{I}³, R_{I}⁹, R_{I}¹⁰ and R_{I}¹¹ independently represent H, -OH or an -O-(C₁-C₆)alkyl group;
or alternatively R_{I}² and R_{I}³ and/or R_{I}⁹ and R_{I}¹⁰ and/or R_{I}¹⁰ and R_{I}¹¹ are fused together so as to form an -O-(CH₂)ₙ-O- group linked to the adjacent cycle, wherein n is an integer comprised between 1 and 6;
A represents N, N⁺, N⁺-(C₁-C₆)alkyl or N⁺-benzyl;
B, absent or present, B representing CH, CH₂, C-methyl, C-Benzyl or C-Phenyl when present; D, absent or present, D representing CH or CH₂ when present;
with the proviso that at least one of B and D is present; and
E represents C, CH or CH₂;
F and G both represent either CH or CH₂;
its tautomers, optical and geometrical isomers, racemates, salts, hydrates and mixtures thereof;
and the compound of formula (II) having the following structure: 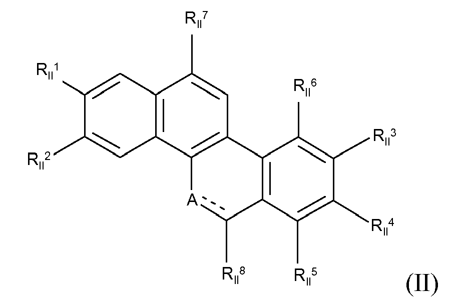 in which
R_{II}¹, R_{II}², R_{II}⁴ and R_{II}⁵ independently represent H, -OH or a -O-(C₁-C₆)alkyl group;
or alternatively wherein R_{II}¹ and R_{II}² and/or R_{II}⁴ and R_{II}⁵ are fused together so as to form an - O-(CH₂)ₙ-O- group linked to the adjacent cycle, wherein n is an integer comprised between 1 and 6;
R_{II}³, R_{II}⁶, R_{II}⁷ and R_{II}⁸ independently represent H, a C₁-C₆ alkyl group, a C₂-C₆ alkylene group or a C₂-C₆ alkynyle group; and
A represents N, N⁺, N⁺-(C₁-C₆)alkyl or N⁺-benzyl;
its tautomers, optical and geometrical isomers, racemates, salts, hydrates and mixtures thereof.

When D is absent in formula (I), member B is present. Compounds according to this definition are compounds of formula (III): 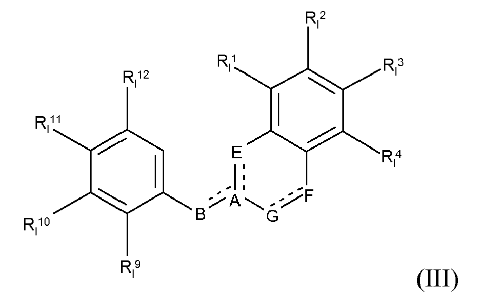 wherein R_{I}¹, R_{I}², R_{I}³, R_{I}⁴, R_{I}⁹, R_{I}¹⁰, R_{I}¹¹, R_{I}¹², A, B, E, F and G are as defined above.

When B is absent in formula (I), member D is present. Compounds according to this definition are compounds of formula (IV): 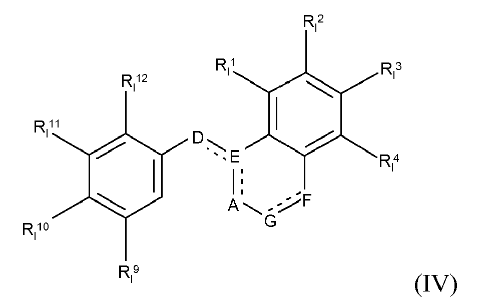 wherein R_{I}¹, R_{I}², R_{I}³, R_{I}⁴, R_{I}⁹, R_{I}¹⁰, R_{I}¹¹, R_{I}¹², A, D, E, F and G are as defined above.

In the structures depicted above, a dotted line denotes the presence or not of a double bond at the indicated position.

When B represents C-Benzyl in formula (I) or (IV), the carbone atom is linked to the methyl group of the benzyl moiety, as illustrated in the following structure:

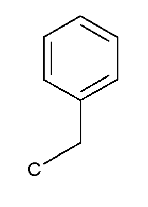

Similarly, when A represents N⁺-benzyl in formula (I), (II), (III) or (IV), the nitrogen atom is linked to the methyl group of the benzyl moiety, as illustrated in the following structure:

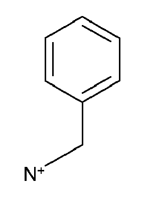

In the present application alkyl, alkenyl and alkynyl groups may be substituted or not by at least one substituent such as halo, amino, cyano, hydroxy, alkoxy, alkylthio, ~NH(alkyl), -NH (cycloalkyl), -N(alkyl)₂, -C(=O)H, -CO₂ H, -CO₂-alkyl, cycloalkyl, substituted cycloalkyl, aryl, heteroaryl, or heterocycle.

Within the context of the present application, the term alkyl denotes linear or branched saturated groups containing from 1 to 6 carbon atoms. Examples of alkyl groups having from 1 to 6 carbon atoms inclusive are methyl, ethyl, propyl, isopropyl, t-butyl, n-butyl, pentyl, hexyl, 2-methylbutyl, 2-methylpentyl and the other isomeric forms thereof. Preferably, the alkyl groups have from 1 to 3 carbon atoms.

The cycloalkyl group is more specifically an alkyl group forming at least one cycle. Examples of cycloalkyl groups having from 3 to 8 carbon atoms inclusive are cyclopropyl, cyclobutyl, cyclopentyl and cyclohexyl. The cycloalkyl group may be optionally substituted.

The heterocycle is understood to refer to hydrocarbon cyclic group having from 1 to 20 carbon atoms, optionally interrupted with one or more heteroatoms selected in the group consisting of N, O, S and P. Among such mono- or poly-cyclic hydrocarbon groups, cyclopentyl, cyclohexyl, cycloheptyl, 1- or 2-adamantyl groups, pyran, piperidine, pyrrolidine, morpholine, dioxan, tetrahydrothiophene, and tetrahydrofuran can be cited.

The term alkenyl denotes linear or branched hydrocarbon groups containing from 2 to 6 carbon atoms and containing at least one double bond. Examples of alkenyl containing from 3 to 6 carbon atoms are 1-propenyl, 2-propenyl, 1-butenyl, 2-butenyl, 3-butenyl, 1-pentenyl, 2-pentenyl, 3-pentenyl, 4-pentenyl, 1-hexenyl, 2-hexenyl, 3-hexenyl, 4-hexenyl, 5-hexenyl and the isomeric forms thereof.

The term alkynyl denotes linear or branched hydrocarbon groups containing from 2 to 6 carbon atoms and containing at least one triple bond. Examples of alkynyl containing from 3 to 6 carbon atoms are 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl and the isomeric forms thereof.

The term aryl includes any aromatic group comprising preferably from 5 to 14 carbon atoms, preferably from 6 to 14 carbon atoms, optionally interrupted by one or several heteroatoms selected from N, O, S or P (termed, more specifically, heteroaryl). Most preferred aryl groups are mono- or bi-cyclic and comprises from 6 to 14 carbon atoms, such as phenyl, α-naphtyl, β-naphtyl, antracenyl, or fluorenyl group.

An alkoxy group denotes an -O-alkyl group and an alkylthio group denotes an -S-alkyl group.

The term "halo" refers to fluorine, chlorine, bromine and iodine.

The in vitro method of the invention may be useful for different purposes. For example, a compound of formula (I) or (II) may be used to modulate, preferably to inhibit, the Rho GTPases, preferably Rac GTPases, in a cell culture for study of the signal pathways involving said GTPases and understanding their biochemical functions or those of their effectors.

In another example, one can use a compound of formula (I) or (II) for modulating, preferably inhibiting, *in vitro* a Rho GTPase, preferably a Rac GTPase, in a screening assay. More specifically, the invention relates to a method for identifying, selecting or characterizing compounds modulating *in vitro* a Rho GTPase, preferably a Rac GTPase, comprising contacting said GTPase with at least one compound of formula (I) or (II) as defined above and with a test compound, and measuring the activity of said GTPase. In a particular embodiment, the method for identifying, selecting or characterizing compounds modulating *in vitro* a Rho GTPase, preferably a Rac GTPase, further comprises comparing the activity of said GTPase in presence of the test compound to the activity of said GTPase in the absence of the test compound. More particularly, the activity of said GTPase can be measured as described below.

In a particular embodiment of the method of the invention, the member of the Rho GTPase family is contacted with a compound of formula (I) in which R_{I}² and/or R_{I}³ represent -OH.

In another particular embodiment of the method of the invention, the member of the Rho GTPase family is contacted with a compound of formula (I) wherein R_{I}² and R_{I}³ and/or R_{I}⁹ and R_{I}¹⁰ and/or R_{I}¹⁰ and R_{I}¹¹ are fused together so as to form an -O-(CH₂)ₙ-O- group linked to the adjacent cycle and preferably wherein n is 1.

In a further particular embodiment of the method of the invention, the member of the Rho GTPase family is contacted with a compound of formula (II), wherein R_{II}¹ and R_{II}² and/or R_{II}⁴ and R_{II}⁵ are fused together so as to form an -O-(CH₂)ₙ-O- group linked to the adjacent cycle and preferably wherein n is 1.

In a particular embodiment of the method of the invention, the member of the Rho GTPase family is contacted with a compound of formula (I) wherein at least one of R_{I}², R_{I}³, R_{I}⁹, R_{I}¹⁰ and R_{I}¹¹ groups represents an -O-(C₁-C₆)alkyl group, preferably an -O-methyl group.

In another particular embodiment of the method of the invention, the member of the Rho GTPase family is contacted with a compound of formula (II) wherein at least one of R_{II}¹, R_{II}², R_{II}⁴ and R_{II}⁵ groups represents an -O-(C₁-C₆)alkyl group, preferably an -O-methyl group.

In a further embodiment of the method of the invention, the member of the Rho GTPase family is contacted with a compound of formula (I) wherein R_{I}¹, R_{I}⁴ and/or R_{I}¹² represent H, preferably wherein R_{I}¹ and R_{I}⁴ represent H and/or R_{I}¹² represents H and more preferably wherein R_{I}¹, R_{I}⁴ and R_{I}¹² simultaneously represent H.

In another particular embodiment of the method of the invention, the member of the Rho GTPase family is contacted with a compound of formula (I) wherein R_{I}¹⁰ represent an - O-methyl or -OH group and optionally R_{I}⁹, or alternatively R_{I}¹¹, represents an -O-methyl or - OH group. In a further embodiment, R_{I}¹⁰ and R_{I}⁹, or alternatively R_{I}¹⁰ and R_{I}¹¹, are the same and preferably represent either an -O-methyl or -OH group.

In another particular embodiment of the method of the invention, the member of the Rho GTPase family is contacted with a compound of formula (I) in which R_{I}², R_{I}³, R_{I}⁹ and/or R_{I}¹⁰ represent -OH.

In a further embodiment of the method of the invention, the member of the Rho GTPase family is contacted with a compound of formula (I) in which R_{I}² and R_{I}³ are -OH and/or R_{I}⁹ and R_{I}¹⁰ are -OH.

In another particular embodiment of the method of the invention, the member of the Rho GTPase family is contacted with a compound of formula (I) in which R_{I}², R_{I}³, R_{I}⁹ and R_{I}¹⁰ represent -OH.

In a particular embodiment of the method of the invention, the member of the Rho GTPase family is contacted with compounds of formula (I) in which R_{I}² and R_{I}³ represent - OH, A is N⁺, B and D represent CH, E represents C and F and G simultaneously represent CH₂.

In a particular embodiment of the invention, when A in the compound of formula (I) or (II) represents N⁺, N⁺-(C₁-C₆)alkyl or N⁺-benzyl, said compound of formula (I) or (II) is an ammonium salt in complex with any suitable counter ion. For example, such compound may be a halide salt such as bromide, chloride, fluoride or iodide salt or an acetate (CH₃-COO⁻) salt.

In a particular embodiment of the method of the invention, the member of the Rho GTPase family, preferably a member of the Rac GTPase family, is contacted with a compound of formula (I) or (II) which inhibits the activity of said member by at least 10 %, preferably at least 20 %, preferably at least 30 %, preferably at least 50 %, preferably at least 60 %, preferably at least 80 %, preferably at least 90 % and more preferably at least 95 % at a concentration of the compound of 50 µM, as determined in the biological assays disclosed below. More specifically, the activity of a member of the Rho GTPase family, preferably a member of the Rac GTPase family, and the effect of a compound of formula (I) or (II) on said GTPase may be determined using an analog of GTP, BODIPY-GTP. The fluorescence of BODIPY-GTP increases when it binds to small G proteins. This property may be used to assess the ability of a compound to modulate the nucleotide binding activity of a GTPase, in particular in a biochemical exchange assay. More particularly, the effect of a compound of formula (I) or (II) may be assessed by determining the binding of BODIPY-GTP to Rac1 activated by the DH/PH domain of Tiam1, to Raclb or to Cdc42.

Specific examples of compounds of formula (I) or (II) which may be used in the above *in vitro* method include the following compounds:
Protoberberine class of isoquinoline alkaloids formula (I)
   Berberine or 1,2-dimethoxy-*N*-methyl-[1,3]benzodioxolo[5,6-*c*]phenanthridinium chloride 1,
   palmatine chloride, hydrate **2**,
   (±)-canadine or (±)-tetrahydroberberine hydrochloride **3,**
   demethyleneberberine or 9,10-dimethoxy-5,6-dihydro-isoquino[3,2-*a*]isoquinolinylium-2,3-diol chloride **4,** (±)-*N*-benzyl canadinium or (±)-7-benzyl-9,10-dimethoxy-5,8,13,13*a*-tetrahydro-6*H-*[1,3]dioxolo[4,5-*g*]isoquino[3,2-*a*]isoquinolinylium bromide **5,** 2,3,9,10-tetrahydroxyberberine or 5,6-dihydro-isoquino[3,2-*a*]isoquinolinylium-2,3,9,10-tetraol chloride **6,**
   2-(2,3-dimethoxybenzyl)-6,7-dimethoxy-1,2,3,4-tetrahydroisoquinoline hydrochloride **7,** coralyne or 8-methyl-2,3,10,11-tetramethoxydibenzo[*a,g*]quinolizinium chloride, hydrate **8,** papaverine or 1-(3,4-dimethoxybenzyl)-6,7-dimethoxyisoquinoline hydrochloride 9, 9,10-dimethoxy-8-phenyl-5,8-dihydro-2*H*-6*H*-[1,3]dioxolo[4,5-*g*]isoquino[3,2-*a*]isoquinoline **10,**
   8-benzyl-9,10-dimethoxy-5,8-dihydro-2*H*-6*H*-[1,3]dioxolo[4,5-*g*]isoquino[3,2-*a*]isoquinoline **11,**
   (±)-8-benzyl-9,10-dimethoxy-5,8,13,13a-tetrahydro-6*H*-[1,3]dioxolo[4,5-*g*]isoquino[3,2-*a*]isoquinoline hydrochloride **12,**
   8-methyl-isoquino[3,2-*a*]isoquinolinylium-2,3,10,11-tetraol chloride **15,**
   (±)-tetrahydroxytetrahydroberberine or (±)-5,8,13,13*a*-tetrahydro-6*H*-isoquino[3,2-*a*]isoquinolinylium-2,3,9,10-tetraol hydrochloride **16.**
Benzo[*c*]phenanthridine alkaloids formula (II)
   Sanguinarine or 13-methyl-[1,3]benzodioxolo[5,6-*c*]-1,3-dioxolo[4,5-*i*]phenanthridinium chloride hydrate 13,
   chelerythrine or 1,2-dimethoxy-*N*-methyl[1,3]benzodioxolo[5,6-*c*]phenanthridinium chloride **14.**

### Protoberberine class of isoquinoline alkaloids:

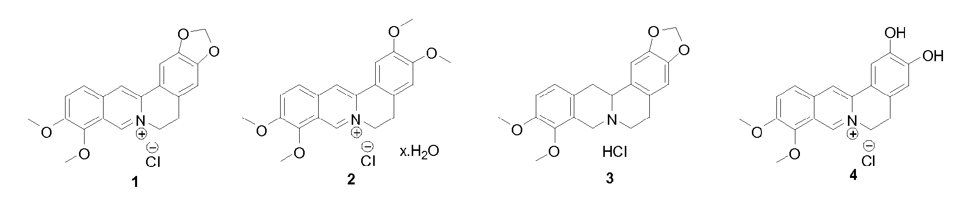

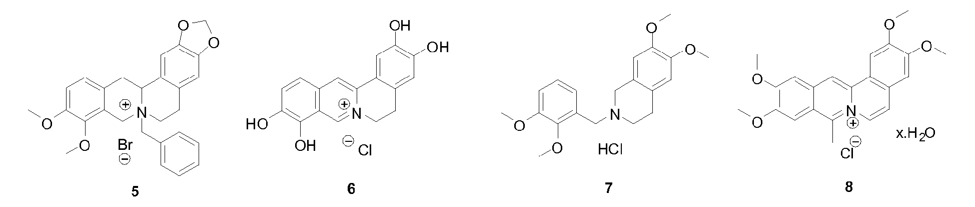

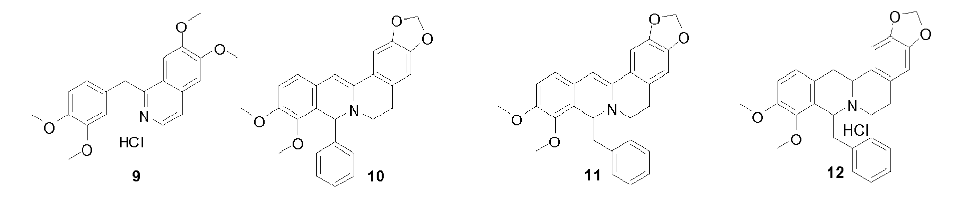

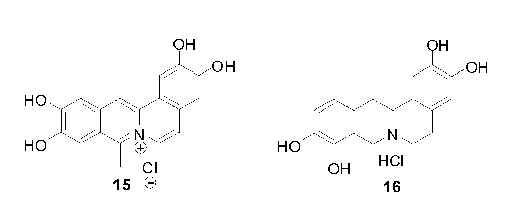

### Benzo[c]phenanthridine alkaloids:

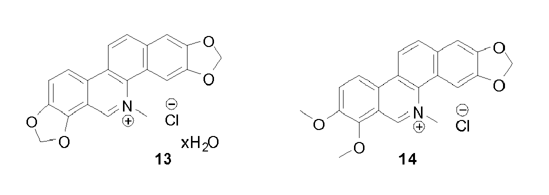

In a particular embodiment, *the in vitro* method of the present invention comprises contacting a member of the Rho GTPase family with a compound of formula **(I)** selected from the group consisting of demethyleneberberine **4,** 2,3,9,10-tetrahydroxyberberine **6** and coralyne hydrochloride **8,** sanguinarine or 13-methyl-[1,3]benzodioxolo[5,6-*c*]-1,3-dioxolo[4,5-*i*]phenanthridinium chloride hydrate **13,** chelerythrine or 1,2-dimethoxy-*N-*methyl[1,3]benzodioxolo[5,6-*c*]phenanthridinium chloride **14,** 8-methyl-isoquino[3,2-*a*]isoquinolinylium-2,3,10,11-tetraol chloride **15,** (±)-tetrahydroxytetrahydroberberine or (±)-5,8,13,13*a*-tetrahydro-6*H*-isoquino[3,2-*a*]isoquinolinylium-2,3,9,10-tetraol hydrochloride **16.**

As indicated above, Rho family proteins constitute one of three major branches of the Ras superfamily. At least 14 mammalian Rho family proteins have been identified so far, including RhoA, RhoB, RhoC, RhoE/Rnd3, Rndl/Rho6, Rnd2/Rho7, RhoG, Rac1, Raclb, Rac2, Rac3, Cdc42, TC10, and TTF.

The present invention discloses experiments showing that compounds of formula (I) or (II) are effective inhibitors of members of the Rho GTPase family, in particular of the Rac GTPase subfamilly, more particularly of Cdc42, Rac1 and/or Raclb.

Accordingly, the invention relates more particularly to *an in vitro* method for modulating, preferably inhibiting, a Rho GTPase selected from the group consisting of RhoA, RhoB, RhoC, RhoE/Rnd3, Rnd1/Rho6, Rnd2/Rho7, RhoG, Rac1, Raclb, Rac2, Rac3, Cdc42, TC10, and TTF. Particularly preferred GTPases are Cdc42, Rac1 and/or Raclb wherein said GTPase is contacted with a compound of formula (I) or (II) as defined above.

According to another embodiment, the invention provides *an in vitro* method for modulating, preferably inhibiting, a Cdc42 or a Rac GTPase, preferably Rac1 and/or Rac1b.

In another particular embodiment, *the in vitro* method of the invention is implemented for modulating, preferably inhibiting, a Rac GTPase selected from the group consisting of Rac1, Raclb, Rac2, Rac3 and mixture thereof. In a further embodiment of the method of the invention, the Rac GTPase is selected from the group consisting of Rac1, Raclb and mixture thereof.

In a further embodiment of the invention, the method of the invention comprises contacting a compound of formula (II), preferably sanguinarine **13** or chelerythrine **14,** with Raclb.

Another object of the invention provides a compound of formula (I) or (II) as defined above. In a particular embodiment, the invention relates to compound 8-methyl-isoquino[3,2-*a*]isoquinolinylium-2,3,10,11-tetraol chloride **15.**

Another object of the invention relates to a compound of formula (I) or (II), as a medicament. In particular, the invention relates to an 8-methyl-isoquino[3,2-*a*]isoquinolinylium-2,3,10,11-tetraol salt, and preferably an halide salt such as 8-methyl-isoquino[3,2-*a*]isoquinolinylium-2,3,10,11-tetraol chloride **15,** as a medicament.

A further object of the invention relates to a pharmaceutical composition comprising at least one compound of formula (I) or formula (II), as defined above, and a pharmaceutically acceptable vehicle or support.

In a preferred embodiment of the invention, the pharmaceutical composition of the invention comprises compound 8-methyl-isoquino[3,2-*a*]isoquinolinylium-2,3,10,11-tetraol salt, and preferably an halide salt, such as 8-methyl-isoquino[3,2-*a*]isoquinolinylium-2,3,10,11-tetraol chloride **15** and a pharmaceutically acceptable vehicle or support.

The compounds may be formulated in various forms, including solid and liquid forms, such as tablets, gel, syrup, powder, aerosol, etc.
The compositions of this invention may contain physiologically acceptable diluents, fillers, lubricants, excipients, solvents, binders, stabilizers, and the like. Diluents that may be used in the compositions include but are not limited to dicalcium phosphate, calcium sulphate, lactose, cellulose, kaolin, mannitol, sodium chloride, dry starch, powdered sugar and for prolonged release tablet-hydroxy propyl methyl cellulose (HPMC). The binders that may be used in the compositions include but are not limited to starch, gelatin and fillers such as sucrose, glucose, dextrose and lactose.
Natural and synthetic gums that may be used in the compositions include but are not limited to sodium alginate, ghatti gum, carboxymethyl cellulose, methyl cellulose, polyvinyl pyrrolidone and veegum. Excipients that may be used in the compositions include but are not limited to microcrystalline cellulose, calcium sulfate, dicalcium phosphate, starch, magnesium stearate, lactose, and sucrose. Stabilizers that may be used include but are not limited to polysaccharides such as acacia, agar, alginic acid, guar gum and tragacanth, amphotsics such as gelatin and synthetic and semi-synthetic polymers such as carbomer resins, cellulose ethers and carboxymethyl chitin.
Solvents that may be used include but are not limited to Ringers solution, water, distilled water, dimethyl sulfoxide to 50% in water, propylene glycol (neat or in water), phosphate buffered saline, balanced salt solution, glycol and other conventional fluids.

The dosages and dosage regimen in which the compounds of formula (I) or (II) are administered will vary according to the dosage form, mode of administration, the condition being treated and particulars of the patient being treated. Accordingly, optimal therapeutic concentrations will be best determined at the time and place through routine experimentation.

The compounds of formula (I) or (II) can also be used enterally. Orally, the compounds according to the invention are suitable administered at the rate of 100 µg to 100 mg per day per kg of body weight. The required dose can be administered in one or more portions. For oral administration, suitable forms are, for example, tablets, gel, aerosols, pills, dragees, syrups, suspensions, emulsions, solutions, powders and granules; a preferred method of administration consists in using a suitable form containing from 1 mg to about 500 mg of active substance.
The compounds according to the invention can also be administered parenterally in the form of solutions or suspensions for intravenous or intramuscular perfusions or injections. In that case, the compounds according to the invention are generally administered at the rate of about 10 µg to 10 mg per day per kg of body weight; a preferred method of administration consists of using solutions or suspensions containing approximately from 0.01 mg to 1 mg of active substance per ml.

Another object of the invention relates to the use of a compound of formula (I) or (II) as defined above for the manufacture of a pharmaceutical composition for treating a pathology involving a member of the Rho GTPase family.

In a particular embodiment of the invention, the pathology involving a member of the Rho GTPase family is selected from the group consisting of platelet hyperreactivity, hypertension, atherosclerosis, restenosis, cerebral ischemia, cerebral vasospasm, neurodegenerative pathologies, spinal cord injury, cancer of the breast, colon, prostate, ovaries, brain or lung, thrombotic disorders, asthma, glaucoma, osteoporosis and erectile dysfunction.

In a particular embodiment, the disease associated with Rho GTPase activity, preferably Rac GTPases activity, is selected from cancer and neurodegenerative pathologies.

Preferred compounds for use according to the invention include any sub-group as defined above and any specific compounds as identified above.

Another object of the invention is a compound of formula (I) or (II) as defined above for the treatment of a pathology involving a member of the Rho GTPase family as defined above.

A further object of the invention is a method for the treatment of a pathology involving a member of the Rho GTPase family, comprising administering to a patient in need of such treatment an effective amount of at least one compound of general formula (I) or (II) as described above.

"Treatment" or "treating" includes both therapeutic and prophylactic treatments. Accordingly, the compounds may be used at very early stages of a disease, or before early onset, or after significant progression, including metastasis. The term "treatment" or "treating" designates in particular a reduction of the burden in a patient, such as a reduction in cell proliferation rate, a destruction of diseased proliferative cells, a reduction of tumor mass or tumor size, a delaying of tumor progression, as well as a complete tumor suppression.

The compounds may be administered according to various routes, typically by injection, such as local or systemic injection(s). Intratumoral injections are preferred for treating existing cancers. However, other administration routes may be used as well, such as intramuscular, intravenous, intradermic, subcutaneous, etc. Furthermore, repeated injections may be performed, if needed, although it is believed that limited injections will be needed in view of the efficacy of the compounds.

Further aspects and advantages of this invention will be disclosed in the following examples, which should be regarded as illustrative and not limiting the scope of this application.

### BRIEF DESCRIPTION OF THE DRAWINGS

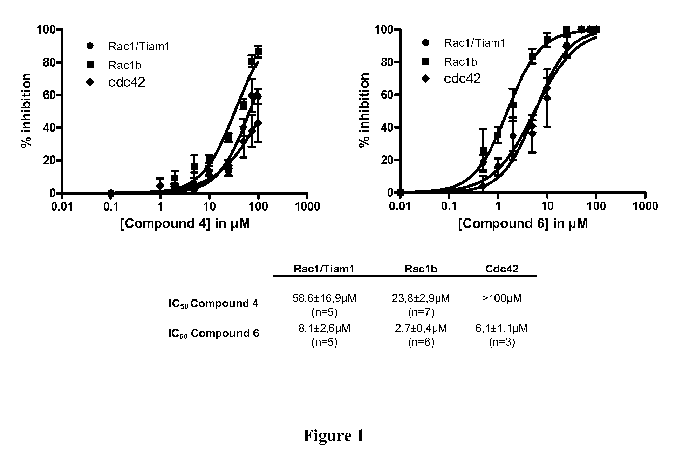

Figure 1: IC₅₀s for protoberberine derivatives
Figure 2: IC₅₀s for protoberberine derivatives
Figure 3: IC₅₀s for benzo[*c*]phenanthridine alkaloids
Figure 4: Dose-response study for control compound NSC 23766

### EXAMPLES

Berberine chloride **1,** palmatine chloride hydrate **2,** papaverine or 1-(3,4-dimethoxybenzyl)-6,7-dimethoxyisoquinoline hydrochloride **9,** 9,10-dimethoxy-8-phenyl-5,8-dihydro-2*H*-6*H*-[1,3]dioxolo[4,5-*g*]isoquino[3,2-*a*]isoquino line **10,** 8-benzyl-9,10-dimethoxy-5,8-dihydro-2*H*-6*H*-[1,3]dioxolo[4,5-*g*]isoquino[3,2-*a*]isoquinoline **11,** sanguinarine chloride hydrate **13** and chelerytrine chloride **14** were obtained from Sigma-Aldrich (St. Louis, MO, USA). Coralyne chloride hydrate **8** was obtained from Acros Organics (New Jersey, USA).

Compounds **3** to **6** and **16** were prepared from berberine chloride **1** by the following synthetic routes summarized below:

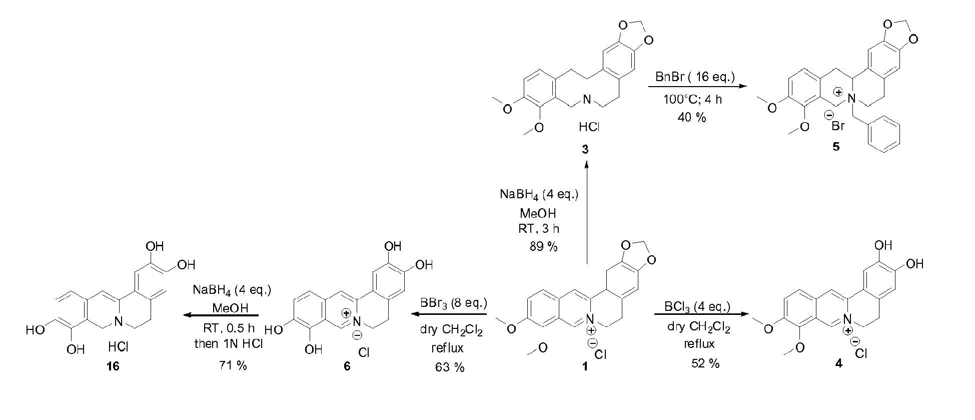

(±)-Canadine hydrochloride **3** was prepared by sodium borohydride reduction of berberine chloride **1** in solution in MeOH (adapted from Ito K., *Yagugaku Zasshi,* 1960, 80, 705) and followed by treatment with an ethanolic HCl solution. (±)-*N*-benzyl canadinium bromide **5** was obtained by reaction of (±)-canadine with an excess of benzyl bromide at reflux for 4 h (adapted from Kametani T., Taguchi E., Yamaki K., Kozuka A., and Terui T., Chem. Pharm. Bull., 1973, 21(5), 1124-1126).

Demethylene berberine chloride **4** was prepared by demethylation of berberine chloride **1** using 4 equivalents of boron trichloride in dichloromethane at reflux (adapted from Hanaoka, M., Nagami, K., Hirai, Y., Sakurai, S.-H., and Yasuda, S., Chem. Pharm. Bull., 1985, 33(6), 2273-2280.). Berberine chloride 1 treated by an excess of boron tribromide in dry dichloromethane at reflux afforded 2,3,9,10-tetrahydroxyberberine chloride **6** (adapted from Colombo M. L., Bugatti, C., Mossa A., Pescalli N., Piazzoni L., Pezzoni G., Menta E., Spinelli S., Johnson F., Gupta R. C., and Dasaradhi L., Il Farmaco, 2001, 56, 403-409.).

(±)-Tetrahydroxytetrahydroberberine hydrochloride **16** was prepared by sodium borohydride reduction of 2,3,9,10-tetrahydroxyberberine chloride **6** in solution in MeOH followed by treatment with 1 N aqueous HCl solution. Compound **16** has also been prepared in the literature by an alternative method (Colombo M. L., Bugatti, C., Mossa A., Pescalli N., Piazzoni L., Pezzoni G., Menta E., Spinelli S., Johnson F., Gupta R. C., and Dasaradhi L., I1 Farmaco, 2001, 56, 403-409.).

Compound **7** was prepared by reductive amination using sodium triacetoxyborohydride, 6,7-dimethoxy-1,2,3,4-tetrahydroisoquinoline hydrochloride and 2,3-dimethoxybenzaldehyde:

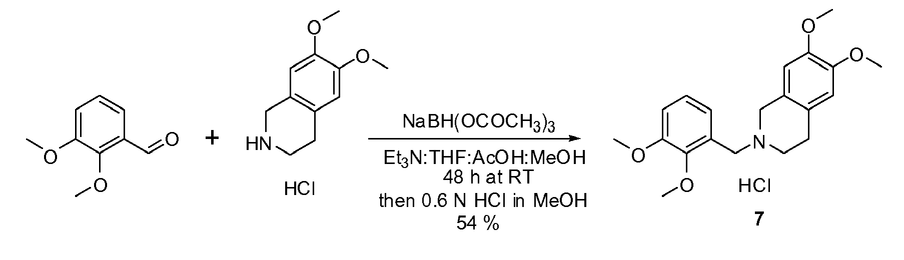

Compound **7** has also been prepared by an alternative method in the literature (Kiparissides, Zinovia et al., Can. J. Chem., 1958, 58, 2770-2779).

Compound **12** was prepared from (±)-8-benzyl-9,10-dimethoxy-5,8,13,13*a*-tetrahydro-6*H*-[1,3]dioxolo[4,5-*g*]isoquino[3,2-*a*]isoquinoline obtained from Sigma-Aldrich (St. Louis, MO, USA). Its hydrochloride salt was purified by preparative HPLC and regenerated as an hydrochloride salt with a methanolic HCl solution.

Coralyne chloride hydrate **8** treated by an excess of boron tribromide in dry dichloromethane for 2 days at RT, followed by 2 N aqueous HCl treatment, afforded to 8-methyl-isoquino[3,2-*a*]isoquinolinylium-2,3,10,11-tetraol chloride **15.** An alternative synthetic preparation of compound 15 has been described in Japanese Patent JP 51034200.

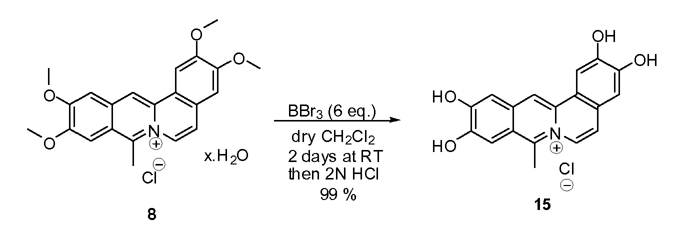

Herein below are presented the origin, synthesis and physico-chemical properties of compounds **1** to **16** according to formula (I) or (II).

### General:

¹H NMR spectra were recorded at ambient temperature with an Advance 300 (Bruker) spectrometer. The compounds were analyzed by reverse phase high performance liquid chromatography (HPLC) using a Waters Autopurification System equipped with a Waters 2525 Pump, a Waters 2696 photodiode array detector, and a XTerra column (Part. No. 186000482, 5 µm, C18, 4.5 x 50 mm). The HPLC method used was a gradient of solvent B:solvent A = 5:95 to 100 % solvent B in 7 min. Solvent A was H₂O with 0.05 % TFA and solvent B was CH₃CN with 0.05 % TFA (Method A). Melting points were measured with a Büchi B-545 melting point apparatus and were uncorrected. To isolate reaction products the solvent were removed by evaporation using a vacuum rotatory evaporator, the water bath temperature not exceeding 40 °C.

### Compounds 1-2, 8-11,13-14:

Berberine chloride **1,** palmatine chloride hydrate **2,** papaverine or 1-(3,4-dimethoxybenzyl)-6,7-dimethoxyisoquinoline hydrochloride **9,** 9,10-dimethoxy-8-phenyl-5,8-dihydro-2*H*-6*H*-[1,3]dioxolo[4,5-*g*]isoquino[3,2-*a*]isoquinoline **10,** 8-benzyl-9,10-dimethoxy-5,8-dihydro-2*H*-6*H*-[1,3]dioxolo[4,5-*g*]isoquino[3,2-*a*]isoquinoline **11,** sanguinarine chloride hydrate **13** and chelerytrine chloride **14** were obtained from Sigma-Aldrich (St. Louis, MO, USA). Coralyne chloride hydrate **8** was obtained from Acros Organics (New Jersey, USA).

### Protoberberine class of isoquinoline alkaloids:

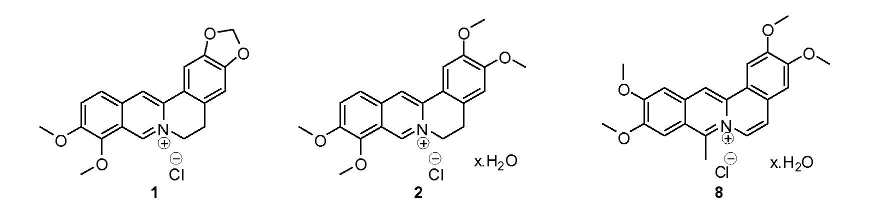

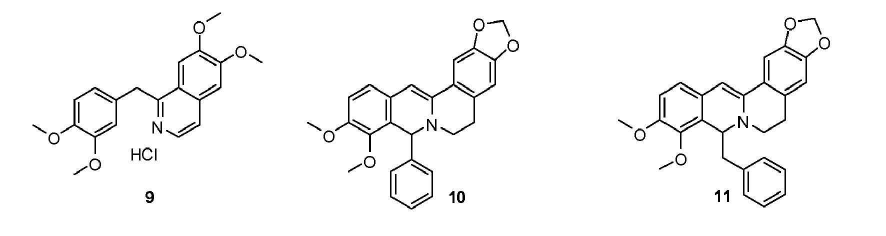

### Benzo[c]phenanthridine alkaloids:

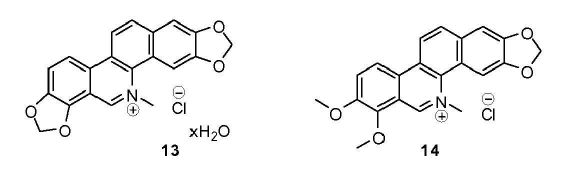

### Preparation of compounds 3-7, 12 and 15-16:

### (±)-Canadine or (±)-tetrahydroberberine hydrochloride 3:

To a solution of berberine chloride (429 mg, 1.15 mmol) in MeOH (35 mL) was slowly added NaBH₄ (174 mg, 4.60 mmol) in a 100 mL round-bottomed flask equipped with a magnetic stirrer. The reaction mixture was stirred at RT for 3 h, after which MeOH was removed at 40°C under vacuum. Water (10 mL) was added and the product was extracted with CH₂Cl₂ (30 mL), then with CH₂Cl₂:MeOH = 5:1 (30 mL). The organic phases were combined, washed with brine (10 mL), dried (Na₂SO₄) and concentrated at 40°C under vacuum, giving 348 mg of a yellow solid (89 % yield). The solid was dissolved in MeOH (10 mL) in a 50 mL round-bottomed flask equipped with a magnetic stirrer and the solution was cooled to 0°C in an ice bath before adding 3.3 mL of a 0.47 N ethanolic HCl solution. The solution was stirred for 15 min at 0°C before concentration to dryness at RT under vacuum. For analysis purpose, a small batch of (±)-canadine hydrochloride **3** was recrystallized in MeOH and the product was isolated as a white solid. 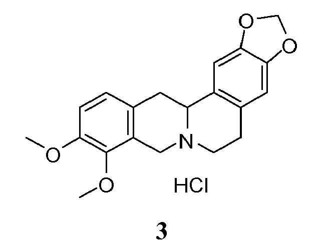 MW: 375.85; Yield: 89 %; White Solid; Mp (°C): 213.5.
*R_{f}*: 0.2 (cyclohexane:EtOAc =4:1, free base).
¹H-NMR (DMSO d₆, δ): 2.85-2.90 (m, 1H, CHH), 3.05 (dd, 1H, *J* = 12.3 Hz & 16.5 Hz, CH*H*), 3.38-3.50 (m, 2H, CH₂), 3.72-3.82 (m, 2H, N-CH₂), 3.79 (s, 3H, O-CH₃), 3.82 (s, 3H, O-CH₃), 4.39 (d, 1H, *J* = 15.3 Hz), 4.66-4.69 (m, 2H), 6.02-6.03 (m, 2H, OCH₂O), 6.83 (s, 1H, Ar-H), 7.01 (d, 1H, *J* = 8.6 Hz, Ar-H), 7.08 (d, 1H, *J*= 8.6 Hz, Ar-H), 7.09 (s, 1H, Ar-H). ¹³C-NMR (DMSO d₆, δ): 25.4, 32.1, 50.0, 51.0, 56.0, 58.9, 60.0, 101.3, 105.6, 108.3, 112.9, 122.5, 124.1, 124.8, 125.1, 125.5, 144.4, 146.7, 146.9, 150.5. MS-ESI *mlz* (rel. int.): 340.0 ([MH]⁺, 100).
HPLC: Method A, detection UV 254 nm, RT = 4.70 min, peak area 96.1 %.

### Demethylene berberine or 9,10-dimethoxy-5,6-dihydro-isoduino[3,2-a]isoduinolinylium-2,3-diol chloride 4:

To a suspension of berberine chloride (6.98 g, 18.8 mmol) in CH₂Cl₂ (155 mL) at O°C under nitrogen in a 500 mL round-bottomed flask equipped with a magnetic stirrer was added dropwise BCl₃ (1 N solution in CH₂Cl₂, 57.6 mL, 57.6 mmol) through a dropping funnel and the reaction mixture was stirred for 1 h at 0°C, then for 20 h at reflux. Another portion of BCl₃ (1 N solution in CH₂Cl₂, 19.0 mL, 19.0 mmol) was then added dropwise to the warm solution and the reaction mixture was stirred overnight at reflux. After cooling to RT, MeOH (100 mL) was carefully added and the volatiles were evaporated at 40°C under vacuum. The solid was then purified by column chromatography (SiO₂; eluent CH₂Cl₂:MeOH = 20:1 to 3:1). The fractions containing the pure product were combined and the solution was concentrated at 40°C under vacuum to a volume of 50 mL. The solution was left to stand for 20 h, after which orange crystals were isolated and identified as demethylene berberine chloride **4** (2.52 g). The filtrate was concentrated to dryness at 40°C under vacuum, affording another batch of **4** (0.97 g). 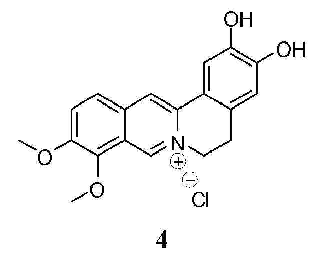 MW: 359.80; Yield: 52 %; Orange Solid; Mp (°C): 219.2.
*R_{f}*: 0.4 (CH₂Cl₂:MeOH = 100:8).
¹H-NMR (CD₃OD, δ): 3.15-3.21 (m, 2H, CH₂), 4.10 (s, 3H, O-CH₃), 4.20 (s, 3H, O-CH₃), 4.87-4.92 (m, 2H, N-CH₂), 6.82 (s, 1H, Ar-H), 7.51 (s, 1H, Ar-H), 7.97 (d, 1H, *J* = 8.4 Hz, Ar-H), 8.09 (d, 1H, *J* = 8.4 Hz, Ar-H), 8.57 (s, 1H, Ar-H), 9.72 (s, 1H, Ar-H).
¹³C-NMR (CD₃OD, δ): 27.7, 57.6, 57.7, 62.5, 113.5, 115.8, 119.5, 120.6, 123.2, 124.4, 128.1, 128.8, 135.5, 140.5, 145.7, 146.2, 147.3, 150.9, 151.7.
MS-ESI *m*/*z* (rel. int.): 324.0 ([M]⁺, 100).
HPLC: Method A, detection UV 254 nm, RT = 4.07 min, peak area 95.9 %.

### (±)-N-benzyl canadinium or (±)-7-benzyl-9,10-dimethoxy-5,8,13,13a-tetrahydro-6H-[1,3]dioxolo[4,5-g]isoquino[3,2-a]isoquinolinylium bromide 5:

A mixture of (±)-canadine (176 mg, 0.52 mmol) and benzyl bromide (1.0 mL, 8.41 mmol) was stirred for 4 h at 100°C in a 10 mL round-bottomed flask equipped with a magnetic stirrer. After cooling, the solid was filtrated, washed several times with Et₂O (5 x 5 mL) and purified by column chromatography (SiO₂; eluent CH₂Cl₂:MeOH = 100:6). (±)-*N-*Benzyl canadinium bromide **5** was isolated as a off-white solid solid (107 mg, 40 % yield). 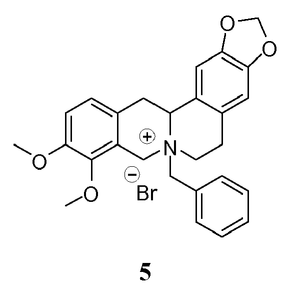 MW: 510.42; Yield: 40 %; Off-white Solid; Mp (°C): 211.5.
*R_{f}*: 0.3 (CH₂Cl₂:MeOH = 100:6)
¹H-NMR (CDCl₃:CD₃OD = 1:1, δ): 3.35-3.41 (m, 2H, CH₂), 3.57-3.70 (m, 2H, CH₂), 3.89 (s, 3H, O-CH₃), 3.97 (s, 3H, O-CH₃), 4.00-4.14 (m, 2H, N-CH₂), 4.19 (s, 2H, N-CH₂), 4.67 (d, 1H, J = 16.0 Hz, N-C*H*H), 4.76 (d, 1H, J = 16.0 Hz, N-CH*H*), 5.53 (dd, 1H, J = 5.6 Hz & 12.1 Hz, N-CH), 6.04-6.05 (m, 2H, OCH₂O) 6.86 (s, 1H, Ar-H), 6.96 (s, 1H, Ar-H), 7.14 (d, 1H, J= 8.7 Hz, Ar-H), 7.21-7.24 (m, 3H, Ar-H), 7.47-7.58 (m, 3H, Ar-H). ¹³C-NMR (CDCl₃:CD₃OD = 1:1, δ): 27.1, 31.7, 54.5, 58.7, 59.2, 59.8, 63.7, 70.1, 104.7, 108.4, 111.4, 116.8, 122.6, 124.7, 125.0, 126.2, 127.5, 128.6, 132.5 (2xC), 134.0, 135.1 (2xC), 147.8, 151.0, 151.5, 154.3.
MS-ESI *m*/*z* (rel. int.): 430.1 ([MH]⁺, 100).
HPLC: Method A, detection UV 254 nm, RT = 5.00 min, peak area 96.5 %.

### 2,3,9,10-Tetrahydroxyberberine or 5,6-dihydro-isoquino[3,2-a]isoquinolinylium-2,3,9,10-tetraol chloride 6:

To a suspension of berberine chloride (1.21 g, 3.25 mmol) in CH₂Cl₂ (55 mL) at -10°C in a 250 mL round-bottomed flask equipped with a magnetic stirrer was added dropwise BBr₃ (1 N solution in CH₂Cl₂, 16.0 mL, 16.0 mmol) and the reaction mixture was stirred for 1 h at - 10°C, then for 17 h at RT. A further portion of BBr₃ (1 N solution in CH₂Cl₂, 10.0 mL, 10.0 mmol) was then added at RT and the reaction mixture was refluxed for a further 6 h, after which it was cooled down to RT, quenched with MeOH (40 mL) and concentrated at 40°C under vacuum. The solid was recrystallized in MeOH to give 2,3,9,10-tetrahydroxyberberine chloride 6 as a yellow solid (682 mg, 63 % yield). 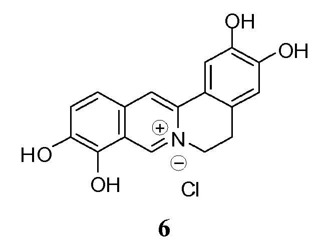 MW: 331.75; Yield: 63 %; Yellow Solid; Mp (°C): 338.8. *R_{f}*: 0.3 (CH₂Cl₂:MeOH = 100:17).
¹H-NMR (CD₃OD, δ): 3.15 (t, 2H, J = 6.2 Hz, CH₂), 4.77-4.90 (m, 2H, N-CH₂), 6.80 (s, 1H, Ar-H), 7.44 (s, 1H, Ar-H), 7.57 (d, 1H, J = 8.2 Hz, Ar-H), 7.74 (d, 1H, J = 8.2 Hz, Ar-H), 8.38 (s, 1H, Ar-H), 9.63 (s, 1H, Ar-H).
¹³C-NMR (CD₃OD, δ): 26.6, 55.9, 111.7, 114.4, 117.8, 118.3, 118.4, 119.1, 126.8, 128.9, 132.8, 137.2, 142.0, 143.6, 143.8, 145.7, 148.9.
MS-ESI *m*/*z* (rel. int.): 296 ([MH]⁺, 100).
HPLC: Method A, detection UV 254 nm, RT = 3.80 min, peak area 99.3 %.

### 2-(2,3-Dimethoxybenzyl)-6,7-dimethoxy-1,2,3,4-tetrahydroisoquinoline hydrochloride 7:

To a solution of 6,7-dimethoxy-1,2,3,4-tetrahydroisoquinoline hydrochloride (2.0 g, 8.70 mmol) and triethylamine (1.2 mL, 17.4 mmol) in THF (120 mL) was added 2,3-dimethoxybenzaldehyde (1.6 g, 7.9 mmol) in a 250 mL round-bottomed flask equipped with a magnetic stirrer. The reaction mixture was stirred for 2 h at 70°C, then at 20°C. MeOH (10 mL), AcOH (0.5 mL) and sodium triacetoxyborohydride (2.5 g, 11.8 mmol) were successively added and the reaction mixture was stirred at RT for 48 h. Water (5 mL) was added, solvents were evaporated and the crude product was partitioned between EtOAc (350 mL) and a 1 M K₂CO₃ solution (50 mL). The organic phase was washed by water (20 mL), brine (20 ml) and was evaporated to give 3.7 g of a crude pale yellow solid. A portion of crude compound (1.0 g) was purified by column chromatography (SiO₂; eluent cyclohexane:EtOAc = 92:8 to 68:32) to give after evaporation a pale yellow powder (450 mg, 56 % yield). The hydrochloride salt was prepared from a portion of free base (220 mg, 0.64 mmol) using a 0.6 N HCl solution in MeOH (1.6 mL, 0.96 mmol) to give after evaporation and drying 2-(2,3-dimethoxybenzyl)-6,7-dimethoxy-1,2,3,4-tetrahydroisoquinoline hydrochloride 7 (235 mg, 54 % yield) as a white solid. 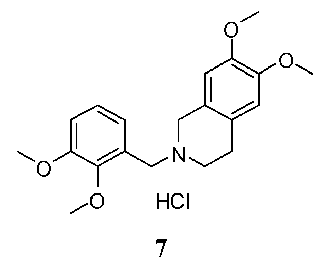 MW: 379.88; Yield: 54 %; White Solid; Mp (°C): 132.5.
*R_{f}:* 0.18 (cyclohexane:EtOAc = 7:3, free base).
¹H-NMR (CD₃OD, δ): 3.00-3.23 (m, 2H, CH₂), 3.35-3.47 (m, 1H, N-CH₂), 3.65-3.77 (m, 1H, N-CH₂), 3.78 (s, 3H, O-CH₃), 3.81 (s, 3H, O-CH₃), 3.91 (s, 3H, O-CH₃), 3.94 (s, 3H, O-CH₃), 4.32 (s, 2H, N-CH₂), 4.47 (s, 2H, N-CH₂), 6.73 (s, 1H, Ar-H), 6.81 (s, 1H, Ar-H), 7.09 (dd, 1H, *J*= 6.2 Hz, *J*= 2.7 Hz, Ar-H), 7.14-7.25 (m, 2H, Ar-H).
¹³C-NMR (CD₃OD, δ): 25.8, 51.0, 53.8, 55.4, 56.4, 56.5, 56.6, 61.6, 110.9, 112.7, 116.3, 120.6, 123.8, 124.3, 124.8, 125.8, 149.8, 150.0, 150.8, 154.3.
MS-ESI *mlz* (rel. int.): 344.0 ([MH]⁺, 100), 150.9 (10).
HPLC: Method A, detection UV 282 nm, RT = 4.3 min, peak area 96.0 %.

### Preparation of compound 12.

### (±)-8-Benzyl-9,10-dimethoxy-5,8,13,13a-tetrahydro-6H-[1,3]dioxolo[4,5-gjisoquino[3,2-a]isoquinoline hydrochloride 12:

(±)-8-Benzyl-9,10-dimethoxy-5,8,13,13*a*-tetrahydro-6*H*-[1,3]dioxolo[4,5-*g*]isoquino[3,2-*a*]isoquinoline (Sigma-Aldrich (St. Louis, MO, USA), 28.8 mg, 0.067 mmol). was dissolved in MeOH (5 mL) in a 10 mL round-bottomed flask equipped with a magnetic stirrer and the solution was cooled to 0°C in an ice bath before adding 0.77 mL of a 0.13 N HCl solution in MeOH. The solution was stirred for 15 min at 0°C before concentration to dryness at RT under vacuum. The solid obtained was dissolved in DMSO (1 mL) and purified using reversed phase HPLC on C18 Xterra Column 19 x 50 mm, 5 µm part 186001108 with a gradient of 0 to 30 % CH₃CN (0.05 % TFA) in H₂O (0.05 % TFA) in 7 min. After 8 injections all the selected fraction were combined and evaporated under reduced pressure to give the desired product (15 mg) which was dissolved in MeOH (1 mL) in a 10 mL round-bottomed flask equipped with a magnetic stirrer and the solution was cooled to 0°C in an ice bath before adding 0.422 mL of a 0.13 N HCl solution in MeOH. After evaporation and drying (±)-8-benzyl-9,10-dimethoxy-5,8,13,13*a*-tetrahydro-6*H*-[1,3]dioxolo[4,5-*g*]isoquino[3,2-*a*]isoquinoline (12 mg, 38 % yield) was obtained as a pale brown solid. 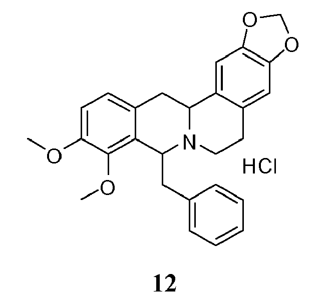 MW: 466.0; Pale Brown Solid; Yield: 38 %; Mp (°C) = 196.9.
¹H-NMR (CD₃OD, δ): 3.05-3.50 (m, 6H, 3xCH₂), 3.60-3.75 (m, 2H, CH₂), 3.90 (s, 3H, OMe), 4.04 (s, 3H, OMe), 4.54 (d, 1H, J= 10.9 Hz, OCH), 5.09 (d, 1H, J = 9.25 Hz, OCH), 5.97 (s, 2H, CH₂), 6.68 (s, 1H, ArH), 7.04 (s, 1H, ArH), 7.12 (s, 2H, ArH), 7.33-7.61 (m, 5H, ArH).
¹³C-NMR (CDCl₃, δ): 27.6, 33.0, 43.0, 54.6, 56.6, 61.2, 64.1, 68.9, 103.1, 106.8, 109.3, 114.6, 125.1, 125.6, 126.3, 126.6, 127.5, 129.0, 129.8 (2xC), 130.6 (2xC), 138.8, 146.6, 149.1, 149.5, 153.1. MS-ESI m/z (% rel. Int.): 430.1 ([MH]⁺, 100).
HPLC: Method A, detection at 280 nm, RT = 6.13 min, peak area 93 %.

### Preparation of compound 15.

### 8-Methyl-isoquino[3,2-a]isoquinolinylium-2,3,10,11-tetraol chloride 15:

To a suspension of coralyne chloride hydrate **8** (723 mg, 1.73 mmol) in CH₂Cl₂ (20 mL) at -78°C in a 250 mL round-bottomed flask equipped with a magnetic stirrer was added dropwise BBr₃ (1 N solution in CH₂Cl₂, 10.5 mL, 10.5 mmol) and the reaction mixture was stirred for 1 h at -78°C, then for 2 days at RT. The reaction medium was cooled down to 0°C in an ice bath, quenched with MeOH (15 mL) and adjusted to pH = 2 with a 2 N aqueous solution of HCl. The volatiles were removed under vacuum at 40°C and the resulting solid was purified by column chromatography (SiO₂; eluent CH₂Cl₂:MeOH = 100:17 to 100:35). 8-Methyl-isoquino[3,2-*a*]isoquinolinylium-2,3,10,11-tetraol chloride **15** was isolated as a yellow solid (0.59 g, 99 % yield). For analysis purpose, a small batch of **15** was purified by preparative HPLC. 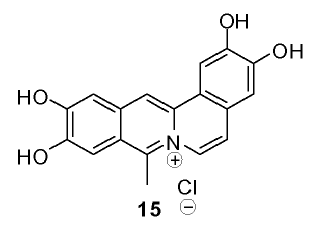 MW: 343.76; Yield: 99 %; Yellow Solid; Mp (°C) > 270 (dec.). *R_{f}*: 0.2 (CH₂Cl₂:MeOH = 100:50).
¹H-NMR (DMSO d₆, δ): 3.21 (s, 3H, CH₃), 7.38 (s, 1H, Ar-H), 7.60 (s, 1H, Ar-H), 7.74-7.77 (m, 1H, Ar-H), 7.82 (s, 1H, Ar-H), 8.26 (s, 1H, Ar-H), 8.55-8.59 (m, 1H, Ar-H), 9.20 (s, 1H, Ar-H), 4 OH not seen. ¹³C-NMR (DMSO d₆, δ): 16.9, 107.2, 107.9, 109.3, 111.5, 114.5, 119.2, 120.2, 121.5, 122.3, 123.2, 133.2, 133.9, 143.9, 149.3, 150.3, 151.5, 155.7.
MS-ESI *m*/*z* (rel. int.): 308 ([M]⁺, 100).
HPLC: Method A, detection UV 254 nm, RT = 3.78 min, peak area 99.9 %.

### Preparation of compound 16.

### (±)-5,8,13,13a-Tetrahydro-6H-isoquino[3,2-a]isoquinolinylium-2,3,9,10-tetraol hydrochloride 16:

To a suspension of 2,3,9,10-tetrahydroxyberberine **6** (350 mg, 1.06 mmol) in MeOH (30 mL) was slowly added NaBH₄ (160 mg, 4.23 mmol) in a 100 mL round-bottomed flask equipped with a magnetic stirrer. The reaction mixture was stirred at RT for 0.5 h, after which the pH was adjusted at 2 with a 1 N aqueous solution of HCl. The volatiles were then removed at 40°C under vacuum. The resulting mixture was taken up in *n*-BuOH (30 mL) and the solution was washed with water (3x15 mL), brine (10 mL), dried (Na₂SO₄) and concentrated at 40°C under vacuum. The solid was then dissolved in hot MeOH (80 mL), precipitated with Et₂O (300 mL) and filtered. The isolated powder was recrystallized from H₂0 to afford (±)-5,8,13,13*a*-tetrahydro-6*H*-isoquino[3,2-*a*]isoquinolinylium-2,3,9,10-tetraol hydrochloride **16** as a brown solid (250 mg ,71 % yield). For analysis purpose, a small batch **16** of was purified by preparative HPLC. 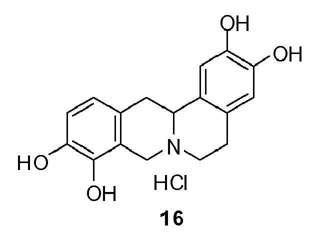 MW: 299.32; Yield: 71 %; Brown Solid; Mp (°C) > 270 (dec.). *R_{f}*: 0.2 (CH₂Cl₂:MeOH = 100:10).
¹H-NMR (DMSO d₆, δ): 2.77 (d, 1H, *J*= 5.0 Hz), 2.90-3.00 (m, 2H), 3.50 (dd, 1H, *J*= 0.8 and 5.0 Hz), 3.77-3.79 (m, 1H), 4.11-4.21 (m, 2H), 4.53 (d, 1H, *J*= 4.7 Hz, Ar-H), 4.52-4.64 (m, 1H), 6.59 (s, 1H, Ar-H), 6.60 (d, 1H, *J* = 2.2 Hz, Ar-H), 6.78 (s, 1H, Ar-H), 6.79 (d, 1H, *J* = 2.2 Hz, Ar-H), 8.99, 9.02, 9.26, 9.47 (4 s, 4H, 4 x OH), 10.86 (br, s, 1H, NH). ¹³C-NMR (DMSO d₆, δ): 24.7, 32.4, 50.4, 51.3, 58.8, 112.3, 115.0, 115.1, 116.4, 118.9, 122.2, 122.5, 122.7, 141.2, 143.1, 144.6, 145.1.
MS-ESI *mlz* (rel. int.): 300 ([MH]⁺, 100).
HPLC: Method A, detection UV 283 nm, RT = 3.33 min, peak area 99.3 %.

### Material and Methods for Biological Assays

### Preparation of recombinant proteins:

The pGEX-Rac1, pGEX-Rac1b, pGEX-Cdc42, pGEX-RhoA and pPRO-Tiam1 DH/PH constructs were kindly provided by C.J. Der (University of North Carolina). BL21 Codon+RIL E. coli strain carrying the constructs of interest is grown in LB medium in presence of 100 µg/ml ampicillin at 37°C 180 rpm during 3 h until optical density reaches 0.6. The expression of recombinant protein is induced by 1 mM IPTG and the culture continued for further 16 h at 20°C.

After centrifugation at 4000g, 20 min 4°C, the bacterial pellet is resuspended in 40 ml/l of culture lysis buffer (50 mM Tris pH 8.6, 300 mM NaCl, 1 mM DTT, 1 µg/ml leupeptine, 1 µg/ml pepstatine, 1 mM benzonase, 2 mM MgCl₂, 50 µg/ml lysozyme and 1 mM EDTA). The solution is incubated 30 min. at 4°C after addition of lysozyme and bacterial lysis is performed by sonication on ice/ethanol. Addition of 1 µM bezonase in lysate is followed by an incubation for 1 h at 4°C with agitation. A centrifugation at 46000 g, 30 min at 4°C allows separating soluble from insoluble proteins.

GST-protein was purified on GST-sepharose 6 Fast Flow (Amersham Bioscience). His6-Tiam1-DH/PH was purified using NI-sepharose 6 Fast Flow (Amersham Bioscience). Pooled fraction from the elution was dialysed against a buffer containing 20 mM Tris pH 8.6, 50 mM NaCl, 1 mM MgCl₂. Aliquots are stored at -80°C in the presence of 10 % glycerol. Protein purifications were done by Protein'eXpert (Grenoble).

### Nucleotide binding assay:

Fluorescence of GTP analog BODIPY-GTP increases when it binds to small G proteins. This property was used to assess ability of compounds to inhibit nucleotide binding to Rac1, Raclb and Cdc42. 2 µM GST-Rac1 and 6 µg His6-Tiam1-DH/PH were diluted in a buffer containing 20 mM Tris, 50 mM NaCl, and 1 mM MgCl₂. This mixture was loaded in a 96-well plate. Fluorescence recording were started (λ_{Ex}: 485 nm ; λ_{Em}: 538 nm) using a spectrofluorimeter (Fluoroskan; Thermolab Systems). Assay was initiated by the addition of 2 µM BODIPY-GTP with or without various doses of test compound. Fluorescence values were recorded, and data were processed as follows: background fluorescence (unbound BODIPY-GTP) was retrieved from fluorescence values. Curves were then analyzed using the GraphPad Prism software which allows performing non-linear regression (One phase exponential association equation: Y = Ymax.(1-e^{-k.X})). Results were expressed as % inhibition = 100 x (Ymax "compound" / Ymax "no compound").

### Results of Biological Assays

Ability of protoberberine derivatives and benzo[c]phenanthridine alkaloids to affect small G proteins activity was studied using a biochemical exchange assay. This assay allows determining whether compounds can inhibit the binding of a fluorescent nucleotide to recombinant small G proteins of interest. The effect of various alkaloid compounds on binding of BODIPY-GTP to Rac1 activated by DH/PH domain of Guanine nucleotide Exchange Factor Tiam1 (Racl/Tiam1), Raclb and Cdc42 is described below. Activity of the compounds is compared to that of NSC 23766 a compound known for its ability to interfere with Rac1 activation by Tiam1.

### Results for protoberberine derivatives:

In total, ten compounds have been tested at 50 µM (see Table 1).
- The compounds showing the highest inhibitory activity on binding of BODIPY-GTP to Rac1/Tiam are 2,3,9,10-tetrahydroxyberberine chloride, compound **6** (100 %), followed by (±)-tetrahydroxytetrahydroberberine hydrochloride, compound **16** (84±6 % inhibition) and 8-methyl-isoquino[3,2-*a*]isoquinolinylium-2,3,10,11-tetraol chloride, compound **15** (67±4 %).
- The compounds showing the highest inhibitory activity on Raclb are compounds 6 (100 %), **15** (80±2 %) and demethyleneberberine chloride **4** (57±3 %).
- Three compounds were tested in the presence of Cdc42: **1** (8±2 %), **4** (31±9 %) and **6** (100 %).

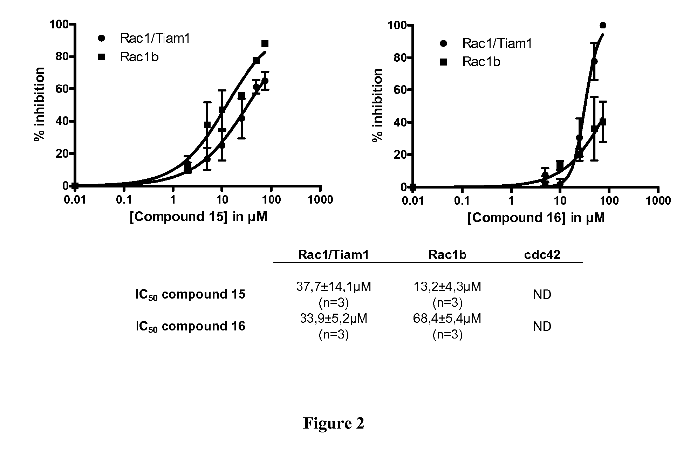

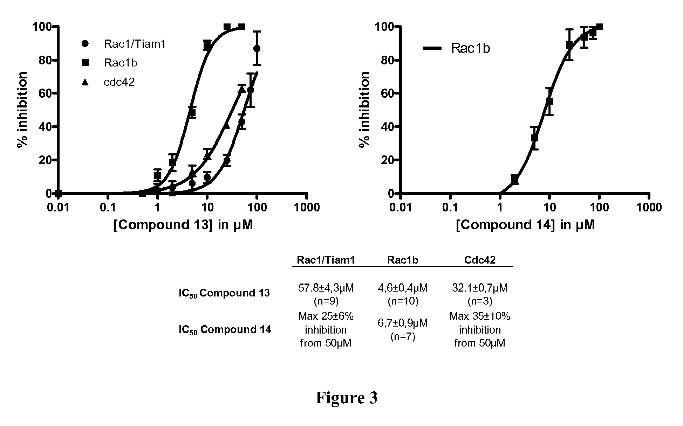

In order to refine these results, dose-response studies were performed on compounds berberine **1,** palmatine **2, 4, 6, 15** and **16** (1-2-5-10-25-50-75-100 µM). For compound **1,** maximum inhibition was of 19±1 % on Rac1/Tiam1 and of 29+1 % on Raclb. For compound **2,** maximum inhibitions were of 17±1 % and 24±2 %, for Rac1/Tiam1 and Raclb, respectively. IC₅₀s could thus not be determined for these compounds. For compounds **4** and **6,** IC₅₀s were calculated and are shown on figure 2. Highest activity compound is compound **6,** with IC₅₀s of 2.7±0.4 µM for Raclb and of 8.1±2.6 µM for Rac1/Tiam1. Compound **4** has IC₅₀s of 23.8±2.9 µM and 58.6±16.9 µM for Raclb and Rac1/Tiam1 respectively. These compounds are about two-fold more active on Raclb than on Rac1/Tiam1. Figure 3 shows dose-response curves obtained for compounds **15** and **16.** Compound **15** is 3-fold more active on Raclb (IC₅₀ of 13.2±4.3 µM) than on Rac1/Tiam1. (±)-Tetrahydroxytetrahydroberberine hydrochloride, compound **16** is the only compound from the protoberberine series that is more potent on Rac1/Tiam1 (IC₅₀ of 33.9±5.2 µM) than on Rac1b (IC₅₀ of 68.4±5.4 µM).

### Results for benzo[c]phenanthridine alkaloids:

In total, two compounds have been tested at 50 µM (see Table 2).
- At 50 µM, sanguinarine chloride **13** has a greater activity against Rac1/Tiam1 (45±4 %) than chelerythrine chloride **14** (25±6 %).
- Both compounds fully inhibit binding of BODIPY-GTP to Raclb at 50 µM.
- Binding of BODIPY-GTP to Cdc42 is inhibited of 63±2 % by compound **13** and of 35±10 % by compound **14.**
- Both compounds seem to display some selectivity that remained to be more clearly defined.

In order to check for compounds selectivity, dose-response studies were performed and IC₅₀s were calculated when possible (see Figure 3).

Sanguinarine **14** is not able to fully inhibit Rac1/Tiam with a max inhibition of around 25 % being observed from 50 µM. A similar effect is observed on Cdc42 (35 % max inhibition from 50 µM). IC₅₀ for Raclb is of 6.7±0.9 µM. Sanguinarine **14** is thus highly selective for Raclb.

Chelerythrine **13** has IC₅₀s of 4.6±0.4 µM for Raclb, 57.8±4.3 µM for Rac1/Tiam1 and 32.1±0.7 µM for Cdc42. It is thus 13 times and 6 times more active on Raclb than on Rac1/Tiam1 and Cdc42 respectively.

As opposed to protoberberine derivatives, benzo[c]phenanthridine alkaloids sanguinarine **13** and chelerythrine **14** showed some significant selectivity for Raclb. Both compounds series would thus be usable for different kinds of pathologies requiring specific or non-specific inhibition of Rac family members.

### Results for control compound NSC 23766:

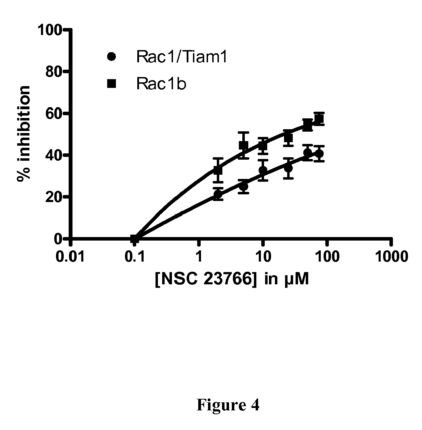

As shown in figure 4, NSC 23766 dose-dependently affects binding of BODIPY-GTP to Rac1/Tiam1 and Rac1b. At maximal dose, NSC 23766 inhibits Rac1/Tiam1 of 33±5 % and Raclb of 57±5 %.

In conclusion, alkaloid compounds are significantly more active on small G proteins Rac than NSC 23766, a compound extensively described for its Rac inhibitory activity (Akbar H., Cancelas J., Williams D.A., Zheng J., and Zheng Y., Methods Enzymol., 2006, 406, 554-65; Gao Y., Dickerson J.B., Guo F., Zheng J., and Zheng Y., Proc Natl Acad Sci U S A., 2004, 101, 7618-23).

| | | | | | | | | | | | | | | | |
|---|---|---|---|---|---|---|---|---|---|---|---|---|---|---|---|
| **Table 1. Protoberberine Derivatives (Isoquinoline Alkaloids)** 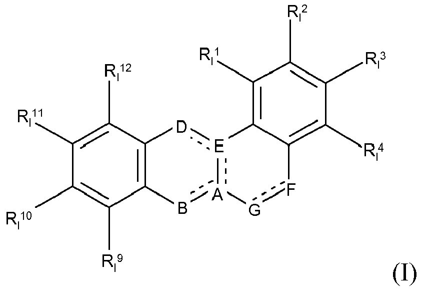 | | | | | | | | | | | | | | | |

| **#** | **Name** | **R_{I}²** | **R_{I}³** | **R_{I}⁹** | **R_{I}¹⁰** | **R_{I}¹¹** | **A** | **B** | **D** | **E** | **F** | **G** | **Rac1** | **Rac1b** | **Cdc42** |
|---|---|---|---|---|---|---|---|---|---|---|---|---|---|---|---|
| **1** | Berberine chloride | OCH₂O | | OMe | OMe | H | N⁺ | CH | CH | C | CH₂ | CH₂ | **15±2** | **23±2** | **8±2** |
| **2** | Palmatine chloride, hydrate | OMe | OMe | OMe | OMe | H | N⁺ | CH | CH | C | CH₂ | CH₂ | **17±2** | **21±3** | ND |
| **3** | (±)-Canadine hydrochloride | OCH₂O | | OMe | OMe | H | N | CH₂ | CH₂ | CH | CH₂ | CH₂ | **1±2** | **0±2** | ND |
| **4** | Demethyleneberberine chloride | OH | OH | OMe | OMe | H | N⁺ | CH | CH | C | CH₂ | CH₂ | **38±3** | **57±3** | **31±9** |
| **5** | (±)-*N*-benzyl canadinium bromide | OCH₂O | | OMe | OMe | H | N⁺Bnz | CH₂ | CH₂ | CH | CH₂ | CH₂ | **12±2** | **19±2** | ND |
| **6** | 2,3,9,10-Tetrahydroxyberberine chloride | OH | OH | OH | OH | H | N⁺ | CH | CH | C | CH₂ | CH₂ | **100** | **100** | 100 |
| **7** | 2-(2,3-Dimethoxybenzyl)-6,7-dimethoxy-1,2,3,4-tetrahydroisoquinoline hydrochloride | OMe | OMe | OMe | OMe | H | N | CH₂ | - | CH₂ | CH₂ | CH₂ | **3±2** | **9±3** | ND |
| **8** | Coralyne chloride, hydrate | OMe | OMe | H | OMe | OMe | N⁺ | CMe | CH | C | CH | CH | **42±13** | **47±12** | ND |
| **9** | Papaverine hydrochloride | OMe | OMe | OMe | OMe | H | N | - | CH₂ | C | CH | CH | **3±2** | **4±2** | ND |
| **10** | 9,10-Dimethoxy-8-phenyl-5,8-dihydro-2*H*-6*H*-[1,3]dioxolo[4,5-*g*]isoquino[3,2-*a*]isoquinoline | OCH₂O | | OMe | OMe | H | N | CPh | CH | C | CH₂ | CH₂ | **13±1** | **26±6** | ND |
| **11** | 8-Benzyl-9,10-dimethoxy-5,8-dihydro-2*H*-6*H*-[1,3]dioxolo[4,5-*g*]isoquino[3,2-*a*]isoquinoline | OCH₂O | | OMe | OMe | H | N | CBnz | CH | C | CH₂ | CH₂ | **2±3** | **-2±6** | ND |
| **12** | (±)-8-Benzyl-9,10-dimethoxy-5,8,13,13*a*-tetrahydro-6*H*-[1,3]dioxolo[4,5-*g*]isoquino[3,2-*a*]isoquinoline HCl | OCH₂O | | OMe | OMe | H | N | CBnz | CH₂ | CH | CH₂ | CH₂ | **22±7** | **41±11** | ND |
| **15** | 8-Methyl-isoquino[3,2-*a*]isoquinolinylium-2,3,10,11-tetraol chloride | OH | OH | H | OH | OH | N⁺ | CMe | CH | C | CH | CH | **67±4** | **80±2** | ND |
| **16** | (±)-Tetrahydroxytetrahydroberberine hydrochloride | OH | OH | OH | OH | H | N | CH₂ | CH₂ | CH | CH₂ | CH₂ | **84±6** | **28±10** | ND |

| | | | | | | | | | | | | | | | |
|---|---|---|---|---|---|---|---|---|---|---|---|---|---|---|---|
| ND: *not determined* *All compounds were tested at a final concentration of 50 µM* | | | | | | | | | | | | | | | |

| | | | | | | | | | | | |
|---|---|---|---|---|---|---|---|---|---|---|---|
| **Table 2. Benzo[*c*]phenanthridine Alkaloids** 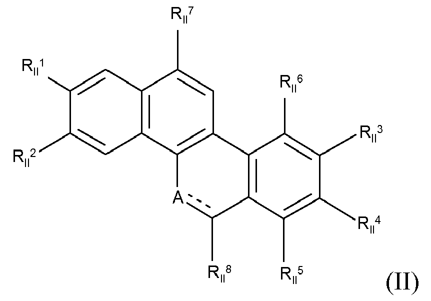 | | | | | | | | | | | |

| **Name** | **R_{II}²** | **R_{II}³** | **R_{II}⁴** | **R_{II}⁵** | **A** | **R_{II}⁶** | **R_{II}⁷** | **R_{II}⁸** | **Rac1** | **Rac1b** | **Cdc42** |
|---|---|---|---|---|---|---|---|---|---|---|---|
| Sanguinarine chloride hydrate **13** | OCH₂O | H | OCH₂O | | N⁺Me | H | H | H | **45±4** | **100** | **63±2** |
| Chelerythrine chloride **14** | OCH₂O | H | OMe | OMe | N⁺Me | H | H | H | **25±6** | **100** | **35±10** |

| | | | | | | | | | | | |
|---|---|---|---|---|---|---|---|---|---|---|---|
| *All compounds were tested at a final concentration of 50 µM* | | | | | | | | | | | |

## Claims

1. An *in vitro* method for inhibiting a member of the Rho GTPase family, wherein the GTPase is contacted with at least one compound of formula (I) or (II), a compound of formula (I) having the following structure: 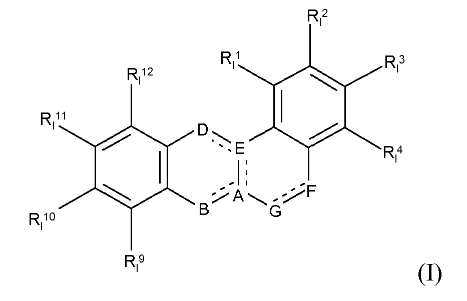 in which
R_{I}¹, R_{I}⁴ and R_{I}¹² independently represent H, a C₁-C₆ alkyl group, a C₂-C₆ alkenyl group or a C₂-C₆ alkynyle group;
R_{I}², R_{I}³, R_{I}⁹, R_{I}¹⁰ and R_{I}¹¹ independently represent H, -OH or an -O-(C₁-C₆)alkyl group;
or alternatively R_{I}² and R_{I}³ and/or R_{I}⁹ and R_{I}¹⁰ and/or R_{I}¹⁰ and R_{I}¹¹ are fused together so as to form an -O-(CH₂)ₙ-O- group linked to the adjacent cycle, wherein n is an integer comprised between 1 and 6;
A represents N, N⁺, N⁺-(C₁-C₆)alkyl or N⁺-arylalkyl;
B, absent or present, represents CH, CH₂, C-Methyl, CH-Methyl, C-Benzyl or C-Phenyl when B is present;
D, absent or present, represents CH or CH₂ when D is present;
with the proviso that at least one of B and D is present; and
E represents C, CH or CH₂;
F and G both represent either CH or CH₂;
its tautomers, optical and geometrical isomers, racemates, salts, hydrates and mixtures thereof;
and the compound of formula (II) having the following structure: 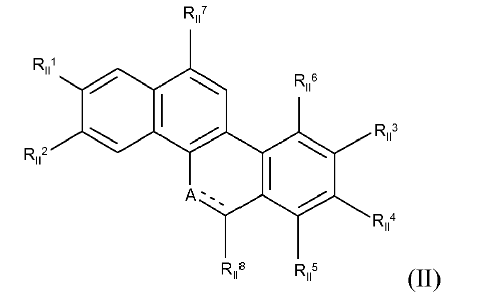 in which
R_{II}¹, R_{II}², R_{II}⁴ and R_{II}⁵ independently represent H, -OH or a -O(C₁-C₆)-alkyl group;
or alternatively wherein R_{II}¹ and R_{II}² and/or R_{II}⁴ and R_{II}⁵ are fused together so as to form an - O-(CH₂)ₙ-O- group linked to the adjacent cycle, wherein n is an integer comprised between 1 and 6;
R_{II}³, R_{II}⁶, R_{II}⁷ and R_{II}⁸ independently represent H, a C₁-C₆ alkyl group, a C₂-C₆ alkylene group or a C₂-C₆ alkynyle group; and
A represents N, N⁺, N⁺-(C₁-C₆)alkyl or N⁺-benzyl;
its tautomers, optical and geometrical isomers, racemates, salts, hydrates and mixtures thereof.

2. The method according to claim 1, for inhibiting Cdc42.

3. The method according to claim 1, for inhibiting a member of the Rac GTPase subfamily of Rho GTPase.

4. The method according to claim 3, for inhibiting Rac1 and/or Rac1b.

5. The method according to anyone of claim 1 to 4, wherein said compound is a compound of formula (I) in which R_{I}² and/or R_{I}³ represent -OH.

6. The method according to anyone of claims 1 to 4, wherein said compound is a compound of formula (I) in which R_{I}² and R_{I}³ represent -OH.

7. The method according to anyone of claims 1 to 6, wherein said compound is a compound of formula (I) in which R_{I}⁹ and R_{I}¹⁰ represent -OH.

8. The method according to anyone of claims 1 to 4, wherein said compound is a compound of formula (I) in which R_{I}² and R_{I}³ represent -OH, A is N⁺, B and D represent CH, E represents C and F and G both represent CH₂.

9. The method according to anyone of claims 1 to 4, wherein said compound is a compound of formula (II) in which R_{II}¹ and R_{II}² and/or R_{II}⁴ and R_{II}⁵ are fused together so as to form an - O-(CH₂)ₙ-O- group linked to the adjacent cycle.

10. The method according to claim 1, wherein said compound is selected from the group consisting of:
Berberine or 1,2-dimethoxy-*N*-methyl-[1,3]benzodioxolo[5,6-*c*]phenanthridinium chloride 1,
palmatine chloride, hydrate **2**,
(±)-canadine or (±)-tetrahydroberberine hydrochloride **3,**
demethyleneberberine or 9,10-dimethoxy-5,6-dihydro-isoquino[3,2-*a*]isoquinolinylium-2,3-diol chloride **4,**
(+)-*N*-benzyl canadinium or (±)-7-benzyl-9,10-dimethoxy-5,8,13,13*a*-tetrahydro-6*H-*[1,3]dioxolo[4,5-*g*]isoquino[3,2-*a*]isoquinolinylium bromide **5,**
2,3,9,10-tetrahydroxyberberine or 5,6-dihydro-isoquino[3,2-*a*]isoquinolinylium-2,3,9,10-tetraol chloride **6,**
2-(2,3-dimethoxybenzyl)-6,7-dimethoxy-1,2,3,4-tetrahydroisoquinoline hydrochloride **7,** coralyne or 8-methyl-2,3,10,11-tetramethoxydibenzo[*a,g*]quinolizinium chloride, hydrate **8,** papaverine or 1-(3,4-dimethoxybenzyl)-6,7-dimethoxyisoquinoline hydrochloride **9,** 9,10-dimethoxy-8-phenyl-5,8-dihydro-2*H*-6*H*-[1,3]dioxolo[4,5-*g*]isoquino[3,2-*a*]isoquinoline **10,**
8-benzyl-9,10-dimethoxy-5,8-dihydro-2*H*-6*H*-[[1,3]dioxolo[4,5-*g*]isoquino[3,2-*a*]isoquinoline **11,**
(±)-8-benzyl-9,10-dimethoxy-5,8,13,13*a*-tetrahydro-6*H*-[1,3]dioxolo[4,5-*g*]isoquino[3,2-*a*]isoquinoline hydrochloride **12,**
sanguinarine or 13-methyl-[1,3]benzodioxolo[5,6-*c*]-1,3-dioxolo[4,5-*i*]phenanthridinium chloride hydrate **13,**
chelerythrine or 1,2-dimethoxy-*N*-methyl[1,3]benzodioxolo[5,6-*c*]phenanthridinium chloride **14,**
8-methyl-isoquino[3,2-*a*]isoquinolinylium-2,3,10,11-tetraol chloride **15,** and (±)-5,8,13,13*a*-tetrahydro-6*H*-isoquino[3,2-*a*]isoquinolinylium-2,3,9,10-tetraol hydrochloride **16.**

11. The method according to claim 10, wherein said compound is selected from the group consisting of:
Demethyleneberberine or 9,10-dimethoxy-5,6-dihydro-isoquino[3,2-*a*]isoquinolinylium-2,3-diol chloride **4,**
2,3,9,10-tetrahydroxyberberine or 5,6-dihydro-isoquino[3,2-*a*]isoquinolinylium-2,3,9,10-tetraol chloride **6,**
coralyne or 8-methyl-2,3,10,11-tetramethoxydibenzo[*a,g*]quinolizinium chloride hydrate **8,** sanguinarine or 13-methyl-[1,3]benzodioxolo[5,6-*c*]-1,3-dioxolo[4,5-*i*]phenanthridinium chloride hydrate **13,**
chelerythrine or 1,2-dimethoxy-*N*-methyl[1,3]benzodioxolo[5,6-*c*]phenanthridinium chloride **14,**
8-methyl-isoquino[3,2-*a*]isoquinolinylium-2,3,10,11-tetraol chloride **15,** and
(±)-5,8,13,13*a*-tetrahydro-6*H*-isoquino[3,2-*a*]isoquinolinylium-2,3,9,10-tetraol hydrochloride **16.**

12. The method according to claim 1, wherein said member of the Rho GTPase family is Rac1b and said compound is a compound of formula (II).

13. The method according to claim 12, wherein said compound is selected from the group consisting of:
Sanguinarine or 13-methyl-[1,3]benzodioxolo[5,6-*c*]-1,3-dioxolo[4,5-*i*]phenanthridinium chloride hydrate **13,**
chelerythrine or 1,2-dimethoxy-*N*-methyl[1,3]benzodioxolo[5,6-*c*]phenanthridinium chloride **14.**

14. Use of a compound of formula (I) or (II) for the manufacture of a pharmaceutical composition for treating a pathology involving a member of the Rho GTPase family,
wherein the compound of formula (I) has the following structure: 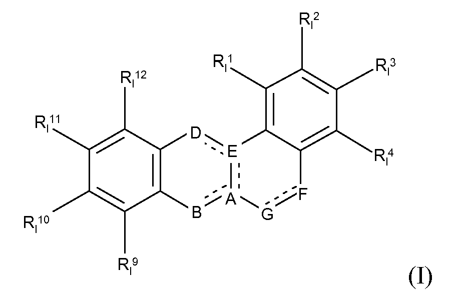 in which
R_{I}¹, R_{I}⁴ and R_{I}¹² independently represent H, a C₁-C₆ alkyl group, a C₂-C₆ alkenyl group or a C₂-C₆ alkynyle group;
R_{I}², R_{I}³, R_{I}⁹, R_{I}¹⁰ and R_{I}¹¹ independently represent H, -OH or an -O-(C₁-C₆)alkyl group;
or alternatively R_{I}² and R_{I}³ and/or R_{I}⁹ and R_{I}¹⁰ and/or R_{I}¹⁰ and R_{I}¹¹ are fused together so as to form an -O-(CH₂)ₙ-O- group linked to the adjacent cycle, wherein n is an integer comprised between 1 and 6;
A represents N, N⁺, N⁺-(C₁-C₆)alkyl or N⁺-arylalkyl;
B, absent or present, represents CH, CH₂, C-Methyl, CH-Methyl, C-Benzyl or C-Phenyl when B is present;
D, absent or present, represents CH or CH₂ when D is present;
with the proviso that at least one of B and D is present; and
E represents C, CH or CH₂;
F and G both represent either CH or CH₂;
its tautomers, optical and geometrical isomers, racemates, salts, hydrates and mixtures thereof;
and wherein the compound of formula (II) has the following structure: 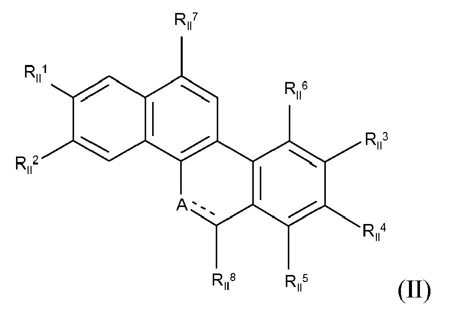 in which
R_{II}¹, R_{II}², R_{II}⁴ and R_{II}⁵ independently represent H, -OH or a -O(C₁-C₆)-alkyl group;
or alternatively wherein R_{II}¹ and R_{II}² and/or R_{II}⁴ and R_{II}⁵ are fused together so as to form an - O-(CH₂)ₙ-O- group linked to the adjacent cycle, wherein n is an integer comprised between 1 and 6;
R_{II}³, R_{II}⁶, R_{II}⁷and R_{II}⁸ independently represent H, a C₁-C₆ alkyl group, a C₂-C₆ alkylene group or a C₂-C₆ alkynyle group; and
A represents N, N⁺, N⁺-(C₁-C₆)alkyl or N⁺-benzyl;
its tautomers, optical and geometrical isomers, racemates, salts, hydrates and mixtures thereof.

15. The use according to claim 14, for inhibiting Cdc42.

16. The use according to claim 14, for inhibiting a member of the Rac GTPase subfamily of Rho GTPase.

17. The use according to claim 16, for inhibiting Rac1 and/or Rac1b.

18. The use according to anyone of claims 14 to 17, wherein said compound is a compound of formula (I) in which R_{I}² and R_{I}³ represent -OH.

19. The use according to anyone of claims 14 to 18, wherein said compound is a compound of formula (I) in which R_{I}⁹ and R_{I}¹⁰ represent -OH.

20. The use according to anyone of claims 14 to 17, wherein said compound is a compound of formula (I) in which R_{I}² and R_{I}³ represent -OH, A is N⁺, B and D represent CH, E represents C and F and G both represent CH₂.

21. The use according to anyone of claims 14 to 17, wherein said compound is a compound of formula (II) in which R_{II}¹ and R_{II}² and/or R_{II}⁴ and R_{II}⁵ are fused together so as to form an -O-(CH₂)ₙ-O- group linked to the adjacent cycle.

22. The use according to claim 14, wherein said compound is selected from the group consisting of:
Berberine or 1,2-dimethoxy-*N*-methyl-[1,3]benzodioxolo[5,6-*c*]phenanthridinium chloride 1,
palmatine chloride, hydrate **2,**
(±)-canadine or (±)-tetrahydroberberine hydrochloride **3,**
demethyleneberberine or 9,10-dimethoxy-5,6-dihydro-isoquino[3,2-*a*]isoquinolinylium-2,3-diol chloride **4,**
(+)-*N*-benzyl canadinium or (±)-7-benzyl-9,10-dimethoxy-5,8,13,13*a*-tetrahydro-6*H-*[1,3]dioxolo[4,5-*g*]isoquino[3,2-*a*]isoquinolinylium bromide **5,**
2,3,9,10-tetrahydroxyberberine or 5,6-dihydro-isoquino[3,2-*a*]isoquinolinylium-2,3,9,10-tetraol chloride **6,**
2-(2,3-dimethoxybenzyl)-6,7-dimethoxy-1,2,3,4-tetrahydroisoquinoline hydrochloride **7,** coralyne or 8-methyl-2,3,10,11-tetramethoxydibenzo[*a,g*]quinolizinium chloride, hydrate **8,** papaverine or 1-(3,4-dimethoxybenzyl)-6,7-dimethoxyisoquinoline hydrochloride **9,** 9,10-dimethoxy-8-phenyl-5,8-dihydro-2*H*-6*H*-[1,3]dioxolo[4,5-*g*]isoquino[3,2-*a*]isoquinoline **10,**
8-benzyl-9,10-dimethoxy-5,8-dihydro-2*H*-6*H*-[[1,3]dioxolo[4,5-*g*]isoquino[3,2-*a*]isoquinoline **11,**
(±)-8-benzyl-9,10-dimethoxy-5,8,13,13*a*-tetrahydro-6*H*-[1,3]dioxolo[4,5-*g*]isoquino[3,2-*a*]isoquinoline hydrochloride **12,**
sanguinarine or 13-methyl-[1,3]benzodioxolo[5,6-*c*]-1,3-dioxolo[4,5-*i*]phenanthridinium chloride hydrate **13,**
chelerythrine or 1,2-dimethoxy-*N*-methyl[1,3]benzodioxolo[5,6-*c*]phenanthridinium chloride chelerythrine or 1,2-dimethoxy-*N*-methyl[1,3]benzodioxolo[5,6-*c*]phenanthridinium chloride **14,**
8-methyl-isoquino[3,2-*a*]isoquinolinylium-2,3,10,11-tetraol chloride **15,** and (±)-5,8,13,13*a*-tetrahydro-6*H*-isoquino[3,2-*a*]isoquinolinylium-2,3,9,10-tetraol hydrochloride **16.**

23. The use according to claim 22, wherein said compound is selected from the group consisting of:
Demethyleneberberine or 9,10-dimethoxy-5,6-dihydro-isoquino[3,2-*a*]isoquinolinylium-2,3-diol chloride **4,**
2,3,9,10-tetrahydroxyberberine or 5,6-dihydro-isoquino[3,2-*a*]isoquinolinylium-2,3,9,10-tetraol chloride **6,**
coralyne or 8-methyl-2,3,10,11-tetramethoxydibenzo[*a,g*]quinolizinium chloride hydrate **8,** 8-methyl-isoquino[3,2-*a*]isoquinolinylium-2,3,10,11-tetraol chloride **15,** and (±)-5,8,13,13*a*-tetrahydro-6*H*-isoquino[3,2-*a*]isoquinolinylium-2,3,9,10-tetraol hydrochloride **16.**

24. A compound of formula (I) 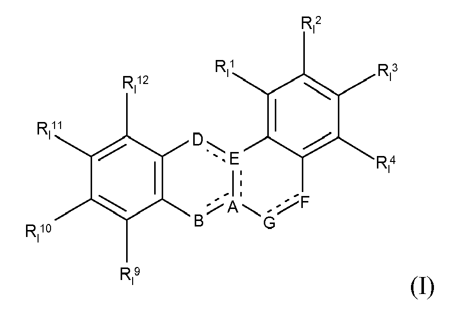 in which
R_{I}¹, R_{I}⁴ and R_{I}¹² independently represent H, a C₁-C₆ alkyl group, a C₂-C₆ alkenyl group or a C₂-C₆ alkynyle group;
R_{I}², R_{I}³, R_{I}⁹, R_{I}¹⁰ and R_{I}¹¹ independently represent H, -OH or an -O-(C₁-C₆)alkyl group;
or alternatively R_{I}² and R_{I}³ and/or R_{I}⁹ and R_{I}¹⁰ and/or R_{I}¹⁰ and R_{I}¹¹ are fused together so as to form an -O-(CH₂)n-O- group linked to the adjacent cycle, wherein n is an integer comprised between 1 and 6;
A represents N, N⁺, N⁺-(C₁-C₆)alkyl or N⁺-arylalkyl;
B, absent or present, represents CH, CH₂, C-Methyl, CH-Methyl, C-Benzyl or C-Phenyl when B is present;
D, absent or present, represents CH or CH₂ when D is present;
with the proviso that at least one of B and D is present; and
E represents C, CH or CH₂;
F and G both represent either CH or CH₂;
its tautomers, optical and geometrical isomers, racemates, salts, hydrates and mixtures thereof.

25. The compound according to claim 24, wherein said compound is 8-methyl-isoquino[3,2-*a*]isoquinolinylium-2,3,10,11-tetraol chloride **15.**

26. A compound of formula (II) 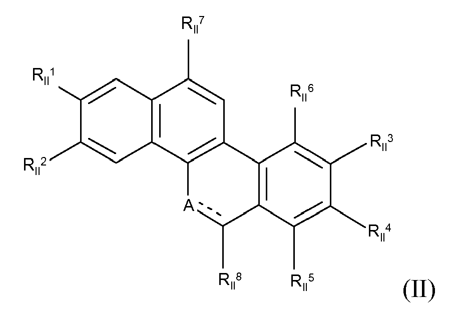 in which
R_{II}¹, R_{II}², R_{II}⁴ and R_{II}⁵ independently represent H, -OH or a -O(C₁-C₆)-alkyl group;
or alternatively wherein R_{II}¹ and R_{II}² and/or R_{II}⁴ and R_{II}⁵ are fused together so as to form an - O-(CH₂)ₙ-O- group linked to the adjacent cycle, wherein n is an integer comprised between 1 and 6;
R_{II}³, R_{II}⁶, R_{II}⁷and R_{II}⁸ independently represent H, a C₁-C₆ alkyl group, a C₂-C₆ alkylene group or a C₂-C₆ alkynyle group; and
A represents N, N⁺, N⁺-(C₁-C₆)alkyl or N⁺-benzyl;
its tautomers, optical and geometrical isomers, racemates, salts, hydrates and mixtures thereof.

27. A pharmaceutical composition comprising at least one compound according to claim 24 or 26 and a pharmaceutically acceptable vehicle or support.
